(19) Europäisches Patentamt
European Patent Office
Office européen des brevets

(11) Publication number: **0 162 891**
**B1**

(12) # EUROPEAN PATENT SPECIFICATION

(45) Date of publication of patent specification: **23.05.90**

(51) Int. Cl.[5]: **C 07 C 2/34,** C 07 C 2/24, C 07 C 45/50, C 07 C 45/45, C 07 C 45/63, C 07 C 209/24, C 07 C 211/08, C 07 C 211/62, C 07 C 239/08

(21) Application number: **84904252.8**

(22) Date of filing: **02.11.84**

(86) International application number:
**PCT/US84/01780**

(87) International publication number:
**WO 85/02173 23.05.85 Gazette 85/12**

(54) **Mixture of amines and process for producing it.**

(30) Priority: **10.11.83 US 551737**
**24.10.84 US 663241**

(43) Date of publication of application:
**04.12.85 Bulletin 85/49**

(45) Publication of the grant of the patent:
**23.05.90 Bulletin 90/21**

(84) Designated Contracting States:
**AT BE CH DE FR GB LI NL SE**

(73) Proprietor: **MONSANTO COMPANY**
**800 North Lindbergh Boulevard**
**St. Louis Missouri 63167 (US)**

(72) Inventor: **FORSTER, Denis**
**32 Woodcrest**
**St. Louis, MO 63124 (US)**

(74) Representative: **McLean, Peter et al**
**Monsanto Europe S.A. Patent Department**
**Avenue de Tervuren 270-272 Letter Box No 1**
**B-1150 Brussels (BE)**

(56) References cited:

| | |
|---|---|
| EP-A-0 033 181 | US-A-3 398 197 |
| WO-A-85/02175 | US-A-3 513 200 |
| CA-A- 747 963 | US-A-3 522 309 |
| DE-A-2 625 945 | US-A-3 597 438 |
| US-A-2 373 705 | US-A-3 729 515 |
| US-A-2 870 207 | US-A-3 819 656 |
| US-A-2 934 568 | US-A-3 947 458 |
| US-A-3 234 283 | US-A-3 976 697 |
| US-A-3 336 387 | US-A-4 032 578 |
| US-A-3 366 583 | US-A-4 139 560 |
| US-A-3 389 178 | US-A-4 143 075 |

(56) References cited:

| | |
|---|---|
| US-A-4 190 601 | US-A-4 346 239 |
| US-A-4 201 725 | US-A-4 388 477 |
| US-A-4 210 605 | US-A-4 400 547 |
| US-A-4 214 998 | US-A-4 404 403 |
| US-A-4 263 324 | US-A-4 421 679 |
| US-A-4 316 990 | US-A-4 426 542 |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 162 891 B1

(56) References cited:
CHEMICAL ABSTRACTS, vol. 79, no. 15, 15th October 1973, page 385, abstract no. 91575m, Columbus, Ohio, US; & JP-A-73 39 451 (KAO SOAP CO., LTD.) 09-06-1973

Chemical Prum., Issued 18 May Jeri Wolf, Hydrogenation Amination of Aldehydes Using Aliphatic Amines

Russian Chemical Reviews, Issued 1980, M.V. Klyuev, Catalylic Amination of Alcohols, Aldehydes and Ketones

## Description

This application is a continuation-in-part of my application S. SN. 551,737, Filed November 10, 1983.

The present invention is concerned with a process for preparing higher alkylated amines from olefin feedstock and with isomeric mixtures of such higher alkylated amines and their amine oxide and quaternary ammonium salt derivatives.

### Background of the Invention

Aliphatic mono-, di and polyamines derived from fatty and rosin acids are reported as a class of surfactants produced in moderate tonnage in the United States in 1980. Primary, secondary, and tertiary monoamines make up the bulk of the class, being sold as acetates, etc. for uses as ore-flotation agents, corrosion inibitors, dispersing agents, wetting agents for asphalt, and as intermediates; see Kirk-Othmer, Encyclopedia of Chem. Technology, Vol. 22, page 378 (Third Edition, 1983, John Wiley and Sons Inc.). The Kirk-Othmer reference (see pages 379, 380) also discusses amine oxides and refers to patents describing their use as surfactants, an amine oxide with a lauryl and two methyl groups being among the many listed.

A number of patents describe particular catalysts or procedures for reductive alkylation of amines with aldehydes, ketones or alcohols, for example, see 3,522,309 which reacts carbonyl compounds selected from aldehydes and ketones having up to 20 carbon atoms, with defined nitrogen compounds selected from ammonia, nitro compounds and primary and secondary amines in the presence of hydrogen and reduction catalyst, and describes an improvement involving use of a polar liquid. However, presently prominent processes for preparation of fatty nitrogen compounds do not employ such alkylations. Thus, a commercial process utilizes a long chain ethylene oligomer which is a substantially linear alpha olefin, hydrobrominates to an alkyl bromide, reacts with dimethylamine to form an ammonium salt, which is then reacted with sodium hydroxide to form the desired tertiary amine and sodium bromide. The process is a mult-step process, and the sodium bromide needs to be recycled from an economics standpoint. Another process proceeds from a long chain, alcohol through an alkyl chloride, also being a multi-step process.

Medium chain length olefins are usually derived from dimerization or oligomerization of ethylene or propylene. Among dimerization processes is the Dimersol® dimerization process for dimerizing olefins using a nickel coordination complex and an aluminum alkyl as catalyst. The process can convert propylene to hexenes with selectivity in excess of 85%. The hexenes can be converted by oxo reaction to aldehydes and then alcohols, producing heptanols. Processes are also known for dimerizing propylene with sodium catalyst to 4-methyl-1-pentene, see Industrial Organic Chemistry, Klaus Weissermel and Hans-Jurgen Arpe; English translation by Alexander Mueller (Verlag Chemie, Weinheim, New York, 1978), pp. 75—77.

US—A—3336387 describes tertiary amine oxides having one long-chain alkyl group which is branched on the beta carbon atom, and mixtures thereof with corresponding amine oxides having only straight-chain alkyl substituents, as components. The amine oxides are made from amines having corresponding branched long-chain alkyl groups or detergents. The branch on the beta carbon atom is a normal primary alkyl group of 1 to 5 carbon atoms and the patent does not describe amines or amine oxides wherein the branch has additional branching.

EP—A—33181 similarly describes amines having a branched alkyl group wherein the branches are linear.

### Summary of the Invention

The present invention concerns a process for preparing amines with long chain alkyl substituents from lower olefins. In the process the olefins are reacted in an oxo process to form aldehydes; the aldehydes are reacted in an aldol reaction to form aldol products which are higher aldehydes, usually in enal form; and the higher aldehydes are reacted with amines to alkylate the amines, producing an amine with an alkyl substituent corresponding to the higher aldehyde reactant, and in very high yield. The process is particularly useful for converting Dimersol® hexenes to $C_{14}$ amines, the Dimersol® hexenes being produced by the dimerization of propylene over trialkyl aluminum and a nickel salt. Thus a facile route from propylene to $C_{14}$ amines is provided. The hexenes, mostly methylpentenes, react in the oxo process to give a highly aldolable aldehyde product which is converted in good yield to $C_{14}$ enals. The enals react readily with primary or secondary amines under reductive conditions to produce dialkylated or monoalkylated products of the amine reactant. The alkyl groups are characterized by branching, being a main branch at the 2-position resulting from the aldol reaction, and generally one or more other branches, usually methyl or ethyl groups, resulting from the oxo reaction or original branched olefin reactant. The amines and their oxide derivatives have potential use in detergent or other surfactant uses, and their quaternary salt derivatives have potential use as germicides.

The composition of the present invention is accordingly one comprising an amine having a higher branched-chain alkyl group attached to the amino nitrogen, said branched alkyl group having 10 to 18 carbon atoms and an alkyl branch containing 3 to 7 carbon atoms at the 2-position, characterised in that (a) the composition comprises a mixture of such amines, (b) most of the amines have additional branching in said higher alkyl group and (c) most of the additional branches are methyl or ethyl groups.

The process of the invention is one for producing a mixture of amines having an N-alkyl substituent with 10 to 18 carbon atoms from olefin feedstock, which comprises reacting an olefin of 4 to 8 carbon atoms

with carbon monoxide and hydrogen to obtain aldehydes having 5 to 9 carbon atoms, subjecting such aldehydes to a base-catalyzed aldol reaction to produce aldol produce aldehydes of 10 to 18 carbon atoms, and reacting such aldol product aldehydes with an amine or ammonia under reductive alkylation conditions to produce amines with an N-alkyl substituent of 10 to 18 carbon atoms.

The aldol stage of the present process produces aldehydes with at least a main branch at the 2-position, and generally an enal form. It has been found that such aldehydes react very readily with amines, particularly in the presence of hydrogen and palladium on carbon catalyst, under very mild conditions, to give very high yields of tertiary amines.

In the present invention it is recognized that detergents or other surfactants with branched hydrophobic structures can have acceptable properties, including biodegradability, despite the general inclination in the art to seek linear or substantially linear structures. With this recognition, it can be seen to be very advantageous to use a preparation process which is a very efficient route for converting a low cost olefin source to detergent range amines, but which results in an amine with branched structure as described herein. In the present process, an olefin can be converted in good yield to aldehydes, with a high percentage of aldolable aldehyde; the aldehydes can be converted to higher aldehydes in good yield by an aldol reaction; and the higher aldehydes are converted to high yield to amines.

The invention further concerns the branched amine compounds produced in the present invention, and amine oxide and quatenary ammonium salt derivatives of the amine compounds.

Detailed disclosure

Catalysts which can be used in reductive alkylation include any catalysts generally used in the reductive alkylation of amines, including noble metals such as platinum or palladium; platinum on carbon or palladium on carbon; nickel catalysts, including Raney nickel, silica on alumina and copper on alumina; silica, titania, zirconia, chromium oxides, copper oxides, molybdenum oxides and mixtures of the foregoing. The catalysts will be used in small but effective amounts, often with 0.1% to 1% by weight of active catalyst material, based on aldehyde reactant, and with the amount of catalyst carrier possibly constituting 1 to 10% of the aldehyde reactant, when the catalyst is employed with a carrier, as in palladium on carbon. The reductive amination will occur under very mild conditions with appropriate catalysts.

The reductive alkylation of an amine by reaction with an aldehyde can be carried out under mild conditions, such as ambient to slightly elevated temperature and moderate hydrogen pressure, such as about 50 up to 1000 or 2000 psi (about 344.7 up to 6895 or 13790 KPa). The use of mild conditions is advantageous for the general convenience and economy of the process, and in addition may contribute to selectivity by avoiding promotion of side reactions which may be accelerated by more rigorous conditions, particularly by higher temperatures. While it will be preferred to carry out the alkylation under mild conditions, and with particularly effective catalysts, such as palladium on carbon, the reaction can be effected at higher temperatures, as high as 100°C on up to 150° or 200°C or so.

The reductive alkylation of amines is also often called the reductive amination of aldehydes, and both terminologies are used herein.

The invention is concerned with a process in which olefins selected from those with 4 to 8 carbon atoms are converted to aldehydes with 5 to 9 carbon atoms, which are then subjected to aldol condensation to obtain aldol products in relatively high yield. The aldehydes can be reacted with amines to produce amines with long chain alkyl substituents with special interest in conversion of hexenes to heptanals and to $C_{14}$ alkyl substituted amines. The invention is further concerned with the foregoing process in which the olefins have 5 to 7 carbon atoms and are converted to aldehydes with 6 to 8 carbon atoms, and then to enals and saturated aldehydes with 12 to 16 carbon atoms, in the detergent hydrophob range, for conversion to amines. Such processes and the aldehyde, amine and detergent derivative products thereof are particularly exemplified herein by processes leading to, and products mainly composed of, amines with 14 carbon atom alkyl groups substituted on the nitrogen. The conditions of the exemplary process are generally applicable to those employing other reactants in the stated carbon atom ranges.

The reductive amination reactions herein can in general be conducted under conditions and with catalysts heretofore known; for example, catalysts for reductive amination of aldehydes among other types of compounds, see Klyuev et al, Russian Chem. Reviews, 49, (1980) pp. 14—27. However, the present aminations involve 2-alkenals with a 2-alkyl branch, and such enals have been found very reactive in aminations, making it feasible to conduct the aminations with good rates under very mild conditions, particularly at mild temperatures, such as ambient temperatures or other mild temperatures not over 50 to 60°C or so. The use of mild conditions aids in avoiding side reactions. Also the reactions can be conducted without resorting to specialized procedures, such as incremental aldehyde addition to keep its concentration low, in order to avoid the aldol reaction. Actually fairly vigorous conditions could be employed with the present enals, with little concern over an aldol reaction since the enals have a 2-substituent and are not prone to aldol reactions. It is further of interest that in the present amination reactions, there was no evidence of addition of the amines across the carbon-to-carbon double bond of the enals. Thus there was apparently no interference from this reaction, even though the double bond is conjugated with the carbonyl bond.

With further regard to the amination reaction, the present aldehydes can be aminated by reaction with ammonia, as well as by reaction with primary or secondary amines. The aminations with ammonia can be

conducted with liquid ammonia or in solvent systems; for general conditions, see Klyuev et al., cited above. The directing of reactions to produce mono-, di-, or tertiary amines is subject to control by using the aminating agent in excess to limit its alkylation, as in producing primary amines, while using the aldehyde in excess to obtain tertiary amines. Of course, when secondary amines are employed, there is only one position for alkylation to obtain a tertiary amine.

Much of the description herein of oxo and aldol reactions and products has been described in U.S.—A—4,426,542, issued January 17, 1984, and the disclosure of the aforesaid patent is incorporated herein by reference.

The invention is further directed to processes for converting olefins to describe $C_{14}$ substituted amines and alcohols, involving dimerization, oxo, aldol and amine alkylation processes based on propylene. In particular, a process involves conducting an oxo reaction with a hexene mixture comprised mainly of methyl pentenes, as produced by dimerization of propylene, and obtaining a heptanal product in which carbonylation has occurred primarily on terminal carbon so that upwards of 75% of the heptanal isomers are unbranched at the 2-position and capable of reacting directly in aldol reactions under basic conditions; and conducting an aldol reaction of the heptanal product to obtain $C_{14}$ aldehydes which are then reacted with amines to produce $C_{14}$ alkyl substituted amines. The process further includes use of cobalt catalyst in the oxo procedure under conditions to produce high percentages of desired aldolable product, and utilization of a co-solvent and suitable aldol conditions to obtain the $C_{14}$ product. Fortuitously, in the hydroformylation of isomeric hexenes, the formylation of the prominent 2-methyl-2-pentene isomer occurs primarily on a terminal bond, with the percentage of such terminal formylation being much higher than that generally characteristic of hydroformylation of internal olefins; and a high percentage of aldolable product is obtained from terminal formylation of isomeric hexenes. The present invention involves an efficient process for converting propylene dimers to amines with an N-alkyl group in the detergent hydrophobe range, in high overall yields such as 70% or better, and the amines are suitable for various surfactant uses, having desirable and biodegradation properties. Moreover, the present process provides a much more efficient and economical route to such amine substituents from olefin feedstock than that provided by the routes prominent in present commercial use. This route permits amine surfactants to be built up from low cost propylene through an efficient series of reactions.

The $C_{14}$ amines of the present invention can be represented:

```
R—CHCH2—NR''R'''
   |
   R'
```

in which R is an alkyl group with seven carbon atoms, and R′ is an alkyl group with five carbon atoms, and R″ and R‴ are hydrogen, hydrocarbyl or hydroxyhydrocarbyl groups, usually lower alkyl or hydroxyalkyl. In a high percentage of the amines in the mixture, generally more than 80—85%, R′ is selected from n-pentyl, 3-methylbutyl and 1-methylbutyl, and R is selected from n-heptyl, 3-methylhexyl, 5-methylhexyl, 2-methylhexyl, and 2-ethylpentyl groups. While there will be some variance in the amine mixture with variation in preparation conditions, generally close to 50% of the amine mixture will be composed of amines with an alkyl group selected from 2-(3-methylbutyl)-5-methyl-octyl, 2-(1-methylbutyl)5-methyloctyl and 2-pentyl-5-methyloctyl. Considering the type of branching involved, close to 50% by weight of the amines will have branching not only at the 2-position of the long chain alkyl, but with each branch having the additional methyl branch, i.e. with R′ and R in the above formula being selected from methylbutyl and methylhexyl groups respectively. The amines are also characterized as generally having a limited amount of vicinal substitution, i.e. substitution or branching on adjacent carbon atoms, and little or virtually no disubstituted carbon chain atoms, i.e. quaternary carbon atoms. The lack of quaternary carbon in the alkyl groups of the amines is fortunate in that such carbon is ordinarily resistant to biodegradation.

The described alkylated amines are obtained by reductive amination of aldehydes of the same structure, which can also be termed reductive alkylation of the amines. Such aldehydes are represented by the formula:

```
R—CHCHO
   |
   R'
```

or when still in the enal form:

```
R=C—CHO
  |
  R'
```

where the double bond is to one of the carbons of the R group. In such formulae, R and R′ have the same meanings as described with respect to the amines, except that R in the enal is now alkylidene. For alcohols, enals and saturated aldehydes of twelve carbon atoms, R has six carbon atoms, and R′ has four; and for sixteen carbon atoms R has eight carbon atoms and R′ has six. R and Ŕ will similarly vary for other structures having 10 to 16 carbon atoms, R from 6 to 8 carbon atoms, and R′ from 5 to 6 carbon atoms. As

with the $C_{14}$ amines, the $C_{10}$, $C_{12}$, and $C_{16}$ aldehydes and amines will often have branching not only at the 2-position, but with each branch having an additional methyl or ethyl branch.

The amines of the present invention can be converted to amine oxides by oxidation:

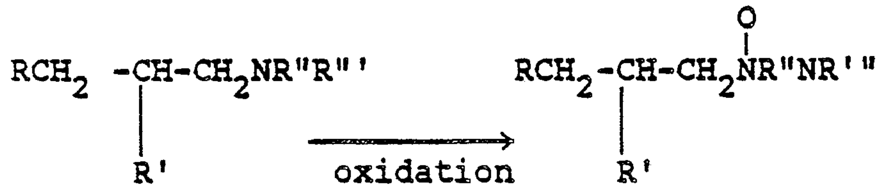


in which the various R, R', R'' and R''' groups have the same meanings as above. The amine oxides are of interest for detergent wetting agent and other surfactant applications. Also, the amines will form quaternary ammonium salts:

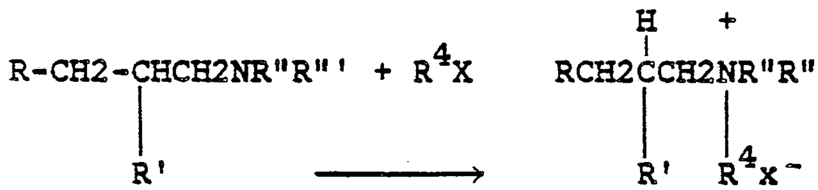

where $R_4$ is an organic radical and X is an anion or anion-forming radical, e.g. halide, and R R', R'' and R''' have the same meanings as above.

A useful group of the amines of the present invention can also be represented:

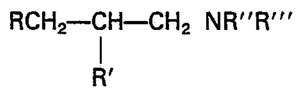

in which R plus R' total 8 to 13 carbon atoms and in which often in a high percentage (75%) of the amines, alkyl branches R and R' can be branched also with branching being limited to methyl or ethyl groups. The R'' and R''' groups are alkyl or hydroxyalkyl groups, generally lower alkyl such as methyl, ethyl, or hydroxyethyl, but can also be $RCH_2CH(R')CH_2$—.

With further regard to the particular $C_{14}$ amine mixture, approximately 40 to 60% by weight of the long chain alkyls will generally be the 2(3-methylbuty)-5-methyloctanyl, 2-(1-methylbutyl)-5-methyloctanyl and 2-pentyl-5-methyloctanyl; and at least about 65% and possibly 65 to 80% or so by weight will have these alkyls, along with 2-(-3methylbuty)-7-methyloctanyl, 2-pentylnonanyl, 2-pentyl-7-methyloctanyl and 2-(3-methylbutyl-nonanyl. In addition to the indicated ranges of these amines, various other $C_{14}$ isomers will be present in small percentages, particularly those with structures of the aldehydes listed in Table 4 herein, in amounts by weight generally no more than 5% each and usually in ranges up to 1 to 2% or so. The long chain alkyls in the mixture are characterized by a fair degree of branching, although the alkyls present in large amounts tend to be less branched than some of the minor components. Thus the 18 alkyl amines constituting about 90—95% by weight of the amines have an average of slightly more than 2 branches in the long alkyl chain, about 2.2 to 2.4 branches, while the other 5 to 10% of the amines may have upwards of above 3.5 such branches, such as 3.75—3.8 branches.

In addition to the novelty of the $C_{14}$ alkyl amine mixtures of the present invention, the particular individual amines may also be unreported.

The amines comprising most of the present mixtures produced from branched hexenes can also be represented by the formula:

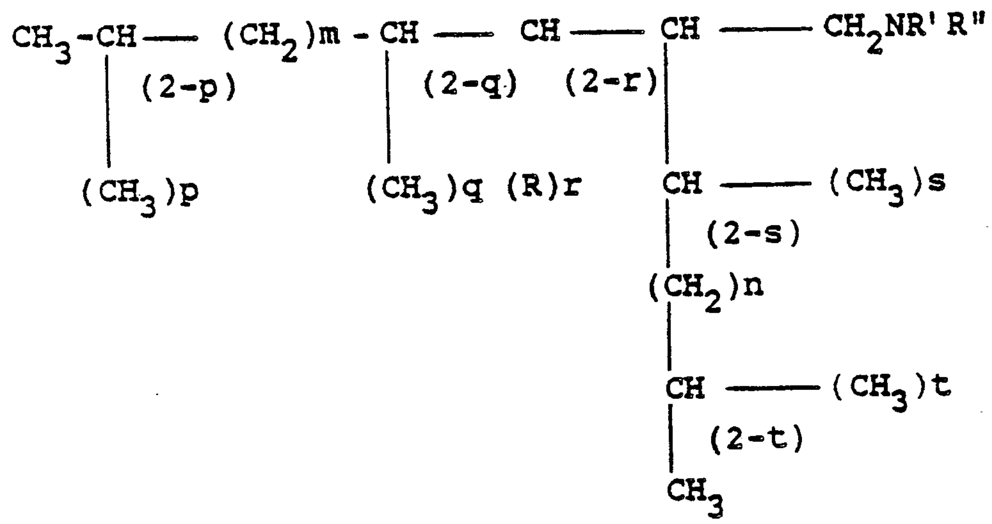

wherein:

R = methyl or ethyl; R′ and R″ are methyl, ethyl, hydroxymethyl or hydroxyethyl;
p, q and r = 0 or 1; but only one of p, q and r can be 1;
m = 3, when p, q and r = 0;
m = 2, when p or q = 1;
m = 2, when r = 1 and R = methyl;
m = 1, when r = 1 and R = ethyl;
n = 2, when s and t = 0;
n = 1, when s or t = 1;
s and t = 0 or 1, but only one of s and t can be 1.

Mixtures of the precursor aldehydes can also be represented by the above formula, with the —$CH_2NR'R''$ group replaced by —CHO. For the corresponding enal structure, there is unsaturation at the 2-position:

$$—CH{=}CH—CHO$$
$$(1-r)$$

In addition to detersive properties, another concern with surfactants has been biodegradability. The first major synthetic detergents were so-called alkylbenzene sulfonates, with an alkyl hydrophobe group derived from propylene tetramer. The propylene tetramer was produced by acid catalyzed polymerization and hence was highly branched, including extensive quaternary branching. This gave rise to a product with rather poor biodegradation properties, which led to extended life for surfactant properties in rivers and lakes. Public concern over the asthetic impact (foam) and possible toxicity of long lasting surfactants led to a voluntary change over to predominantly linear hydrophobes. Prominent among current surfactants are linear alkylbenzene sulfonate and the linear alcohol ethoxylates discussed above. The linear alkylbenzene sulfonate (LAS) involves a linear alkyl group substituted on a benzene ring, generally at one of the secondary carbon atoms of the alkyl group. LAS has been found to be a suitably biodegradable detergent, although being degraded somewhat more slowly in standard tests than the substantially linear alcohol ethoxylates. The branched alkyl amines of the present invention are expected to be suitably biodegradable, possibly comparable in biodegradation to LAS.

Hexenes, as produced by dimerization of propylene with transition metal catalysts, as in the Dimersol® dimerization process, as characterized by being composed mainly of internal olefins, and a linear content which has a range from about 20% up to 32% or so. The main isomer present is a 2-methyl-2-pentene, along with other 2- and 4-methyl pentenes and around 6% 2,3-dimethyl-2-butene.

As indicated above, the linear content of propylene dimers is fairly low, being around 20% to 30% or so. At times there may be advantage in separating the linear components prior to conducting the present process. Separation can be effected by use of a molecular sieve or other suitable procedure. However, it has been found that the entire hexenes portion of the propylene dimerization product can be utilized as feedstock for the present oxo-aldol process. The branched isomers are typified by 2-methyl-2-pentene which, when subjected to oxo reaction with cobalt catalyst, has been found to be very selectively converted to 3-methyl and 5-methylhexanals. Fortunately it has been found that cobalt catalyst, in contrast to rhodium, has the effect of isomerizing the internal olefins so that the aldehyde group is predominantly on the end of the chain. It is very important to the use of propylene dimer in the present process, that the oxo

product is predominantly a non 2-branched alkanal., i.e. there is no substituent in the 2-position. Such aldehydes, which will directly react in base-catalyzed aldol reactions are sometimes referred to herein as "aldolable" aldehydes. As discussed herein, aldehydes with substituents in the 2-position do not readily undergo base-catalyzed aldol reactions. Thus if the internal hexenes were converted largely to such unreactive aldehydes, it would be very difficult to effect self-condensation of such aldehydes to a useful extent, and the use of propylene dimers in the present oxo-aldol process would be impractical. However, as discussed herein, the oxo process with cobalt catalyst converts the hexenes largely, e.g. 75 to 80% or so, to aldehydes which will react in the aldol reaction, making hexenes, obtained from propylene dimerization very suitable as a feedstock for producing detergent range amines in accord with the present invention. This result is surprising in view of the fact that oxo reactions of certain non-terminal octenes are reported to give less than 60% of straight chain aldehyde isomers. It appears that the methyl substituent has some influence in directing the oxo reaction to obtain a great predominance of aldehydes with no substituent in the 2-position. There will similarly be advantage in using branched pentenes and heptenes in the present process, including those with no more than 20 to 30% linear content, even including those with mainly internal unsaturation, and obtain similarly good results in the oxo and aldol reactions taught herein.

Particular branched hexene isomers are converted to non 2-branched alkanals with very high selectivity, with 2-methyl-pentene-1 selectivity of better than 90% to such aldehydes being obtainable. The mixture of both branched and linear hexenes from propylene dimerization can be converted to such aldolable aldehydes with selectivity such as about 79%. In contrast, the selectivity to such aldehydes from the linear hexenes may be only 60% or so. Thus, surprisingly it is found that higher selectivity to aldehydes desirable for the aldol reaction can be obtained by using the crude hexenes mixture for the oxo reaction, rather than only the linear hexenes. Depending upon relative value and availability of the linear and branched hexenes, one might find advantage in using only the branched hexenes in the present process because of the high selectivity in the oxo process to aldehydes with no 2-branching suitable for aldol reaction.

The linear hexenes can be separated from the crude dimerization product by use of a molecular sieve or other suitable procedure, and the linear hexenes alone then subjected to an oxo reaction to obtain heptanal products, with the linear content of the heptanals ranging up to 75% or more. Branched isomers which may be present include 2-methylhexanal, and 2-ethylpentanal. The oxo product mixture can be reacted in an aldol reaction, employing aldol conditions as described herein, to produce aldol products. Under the aldol conditions employed, the n-heptanal reacts with itself at a rate about 10—15 times faster than it reacts with 2-methyl-hexanal or other 2-substituted aldehydes. Thus, when the $C_7$ aldehydes are produced from linear hexenes, a product compound predominantly of product from self-condensation of n-heptanal can be obtained by carrying out an aldol reaction of the oxo reaction product, with some control over the amount of product from 2-substituted aldehydes by controlling the amounts of conversion which is permitted. It may be desirable to have better than 50 to 60% completion for efficient use of feed stock, and conversion of 80% or higher may at times be desirable. The rection can be run to achieve 95% or better conversion of the n-aldehyde to aldol product, while only about 1/4 to 1/3 of the 2-substituted aldehydes are usually converted to aldol product by a cross-aldol reaction. With use of appropriate control, aldol product with about 80% to say 95% from self-condensation of n-heptanal can be obtained, for example at least 85% from self-condensation, with no more than 15% of cross-aldol product. Depending upon the properties desired in the product, the degree of branching can be controlled to a considerable extent by the present process. It happens that some known detergent range alcohols have a fair degree of branching and still have satisfactory biodegradability. Regardless of the desired degree of branching, there is advantage in being able to carry out an aldol reaction on the oxo product of the hexenes, without need for separating branched aldehyde isomers, and obtain useful product, particularly considering the low cost nature of the feed stock and process. With a proper heptanal mixture, the process can be used to obtain a product composed of about 85% 2-pentylnonanal and 15% 2-pentyl-4-methyl-octanal.

The oxo process can generally be utilized to achieve 90—95% yields of aldehydes. With linear hexenes are reactant, selectivity to aldehydes without 2-substituents, i.e. α-aldehydealkanes, can be as high as 60 to 65%, but in large scale operation will possibly range from 50 to 65%.

The present invention in one aspect employs an oxo reaction of substantially branched hexenes to obtain a mixture of aldehydes, which is then subjected to an aldol reaction. The oxo reaction involves contacting hexenes with hydrogen and carbon monoxide and hydroformylation catalyst under hydroformylation conditions suited to obtaining a high proportion of terminal formylation of the olefin feed. It is desirable to have the resulting aldehyde constituted 75 to 80% or more of the aldehyde product.

It is important that the hydroformylation of the mixed hexenes give a relatively high ratio of aldehydes without 2-branching, as this contributes to the feasibility of using the aldehyde mixture for an aldol reaction to obtain a good yield of aldol product. The use of moderate temperatures in the hydroformylation contributes to forming aldehydes without 2-branching, but reaction rate improves with temperature. Thus temperatures sufficient to produce an appreciable reaction rate, ranging from 80° to 100°C can be used, and temperatures on up to 125—140°C can be employed to obtain better reaction rates. Still higher temperatures up to 150°C or higher can be used. To some extent high catalyst concentrations can be employed to obtain reaction rates, even at relatively low temperatures. Cobalt catalyst is especially suited to obtain the desired high proportion of aldehyde with no 2-branches. Unmodified cobalt carbonyl catalyst can

conveniently be used. Such catalyst conventionally designated as dicobalt octacarbonyl, can be provided or employed in many forms known to be useful as a hydroformylation catalyst, although it may be necessary to exercise some choice to provide catalyst best suited to obtaining a high proportion of aldehyde with product suitable for direct base-catalyzed aldol reaction. The above-referred US—A—4426542 disclosed conditions as above for hydroformylation of mixed linear butenes, stating that moderate temperatures in the hydroformylation contribute to obtaining about a 3:1 mixture of normal to branched aldehydes. The conditions can be used for hydroformylation of olefins with 4 to 8, or more narrowly, 5 to 7 carbon atoms. US—A—4 426 542 further described as exemplary a process in which mixed butenes are converted to a ten-carbon plasticizer alcohol comprised of at least about 80—90% 2-propylheptanol by an oxo reaction of the butenes to obtain amyl aldehydes with at least about 66% n-pentaldehyde content, followed by an aldol reaction of the aldehydes under conditions to cause substantially all of the n-pentaldehyde to react but with incomplete conversion of branched aldehydes, and then hydrogenating to produce alcohols in which the ten-carbon alcohols are comprised of at least 80—90% 2-propylheptanol. Under the aldol conditions employed the 2-methylbutanal present does not readily condense with itself, and condenses at a comparatively slow rate with the n-pentanal, so that the 2-propyl-4-methylhexanol content (resulting from the so-called cross aldol of n-pentanal with 2-methylbutanal) in the resulting alcohol is held to no more than about 15—20%, often 12% or less. Under the aldol conditions employed, the n-pentanal reacts with itself to form aldol product at a rate about 15 times greater than it reacts with 2-methylbutanal. The aldol reaction is permitted to go to 80% completion so that if about 25% of the aldehyde supplied is 2-methylbutanal, about 3/4 of it will remain unreacted, and about 88% of the aldol product will be that from self-condensation of n-pentanal. The conversion of n-pentanal to aldol product will be very high and desirably nearly complete, such as upwards of 90 or 95%. There will be some variation with conditions and isomer content of the aldehydes utilized, but it was contemplated to obtain aldol product with about 80% to about 95% being from the self-condensation of n-pentanal, and preferably at least 85% from self-condensation with no more than 15% of 2-propyl-4-methylhexanal being produced. In the process, the aldol intermediate, 2-propyl-3-hydroxyl-heptanal, will ordinarily be dehydrated in the aldol procedure to 2-propylheptenal. Under some conditions the immediate aldol product can be isolated, but ordinarily under the temperature conditions employed the 2-propyl-2-heptenal is produced. In such procedure, it was desirable to have the n-pentanal to branched aldehyde ratio at least about 2.0:1, representing at least about 66.7% n-pentanal content. Aldehydes with 70—75% normal content, or even higher normal contents are desirable to the extent available from oxo reactions, possibly up to 85%, and will be useful for the aldol stage.

The oxo stage of the reaction can be conducted under the usual conditions pertaining to cobalt catalyzed hydroformylation reactions with attention to the temperature conditions as described above. Usual pressure conditions apply, such as 500—4000 or up to 5000 psi (3447.5—27,580 on up to 34,475 kilopascals) total pressure, with most of the pressure being from the carbon monoxide and hydrogen supplied. The carbon monoxide and hydrogen are conveniently used in 1:1 ratio and obtained from usual synthesis gas sources, but other ratios can be employed in keeping with known hydroformylation practice. The reaction can be carried to the desired stage of completion in 1 to 3 hours or so on a batch basis, temperature, pressure and catalyst concentration. While cobalt catalyst is preferred, other hydroformylation catalysts can be used, particularly with appropriate olefin feeds. Migration of double bonds occurs to a great extent in cobalt catalysis, making it very suitable for use with internal olefins. Conversely, rhodium catalyst is well suited for use with α-olefins, giving a very high predominance of terminal hydroformylation.

The reaction can be conveniently conducted either without a solvent or with solvents and, employing concentrations customary for homogeneous catalyst reactions, such as 2 to 10 molar or greater concentrations of the hexenes (or butenes or other olefins) in a solvent, e.g. hydrocarbon solvents such as toluene, and 0.1% to 1% by weight, based on cobalt, of catalyst.

The present invention is particularly concerned with preparing amines with detergent range alkyl substituents from propylene feedstock. The detergent range alcohols are somewhat higher in carbon number than plasticizer alcohols, often having 14 carbon atoms, but in some cases ranging from about 10 or 11 to about 16 carbon atoms. Propylene can be dimerized to hexenes, and the hexenes can be converted to aldehydes by an oxo reaction as described herein, and the resulting heptahaldehydes can be reacted in an aldol reaction to produce aldol products which can be reacted with amines to produce alkylated amines. Using oxo product in which the content of aldehyde without 2-substitution may range from 60 up to near 80%, aldol conversions may approach 75 to 90%, even though participation of the 2-substituted aldehydes is limited, so that it is involved in cross-aldol producing no more than 20% of the product.

The aldol reaction is carried out for the most part utilizing the usual aldol catalysts and temperature conditions, using elevated temperatures upwards of 60°C, particularly temperatures of about 90°C to 130°C, or possibly up to 150°C or higher if desired, the conditions also being those disclosed for n-pentanal in US—A—4 426 542. The reaction is operable over broad pressure ranges including pressures less than atmospheric as well as elevated pressures, but will usually be effected at slightly elevated pressures sufficient to maintain the reactants substantially in the liquid state. The reaction can also conveniently be conducted at reflux.

The aldol reaction can utilize strongly alkaline catalyst, such as sodium and potassium hydroxide, or

sodium and potassium cyanide. The concentration of the aqueous alkali can be varied, but molar or similar concentrations of alkali metal hydroxides can be used, and concentrations selected will often be in the range of about 1 to 10% by weight. The amount of aqueous alkali to aldehyde reactant can also vary, for example from about 15% by volume aqueous alkali up to about 75% by volume aqueous alkali. The aldol reaction will be run for a sufficient time to obtain the desired degree of conversion, which for batch reactions may be in the range of 1 to 3 hours, while in continuous reaction times of less than five minutes are achievable. The reaction is stopped by permitting the reaction mixture to cool and separating the organic reaction phase from the aqueous alkali phase. In some respects there may be advantage in using highly concentrated alkali, such as 5N aqueous alkali, or 4 to 6 N aqueous alkali; e.g. 16 to 24% or so by weight of sodium hydroxide in aqueous solution, which, for reactions of heptanal or other higher aldehydes can be used along with methanol or other co-solvent as dicussed below. The use of such concentrated alkali permits relatively high conversion of the 2-branched aldehyde present in oxo product, such as up to 50% or more. Thus if 80% "aldolable" heptenals are used, conversions of 90% are feasible, and near quantitative selectivities to $C_{14}$ aldehydes can be obtained.

In one major respect it is difficult to conduct aldol reactions of heptanals or other higher aldehydes in conventional manner, as in aqueous NaOH, because this procedure requires appreciable solubility of the organic aldehyde in the aqueous phase, or vice versa. The mutual solubility is so low with $C_7$ aldehydes that little reaction occurs in reasonable time periods. However, if has been found that use of a co-solvent overcomes this problem and results in suitable reaction rates. In principle, any solvent with miscibility with both the aldehyde and the aqueous base, or at least some solubility with respect to each, will act to increase the rate of the reaction. It is also desirable that the solvent be relatively inert under the reaction conditions so as not to cause interfering reactions or to be readily degraded excessively by the hot basic medium. In general, polar solvents will tend to have the requisite solubility characteristics, and hydroxy alkanes, for example, alkane diols, having the appropriate solubility characteristics can be used. Hexanediol is very suitable. Methanol is also a suitable cosolvent.

In the aldol reactions involved in the present invention it is necessary to utilize a high proportion of aldehydes without 2-branching, also referred to herein as aldolable aldehydes, in order to achieve good yields of aldol. The 2-substituted aldehyes do not undergo self-condensation aldol reactions with any facility under the usual basic aldol conditions, although they will serve as acceptor molecules to some extent in cross-aldol reactions, as illustrated with the following heptanal isomers:

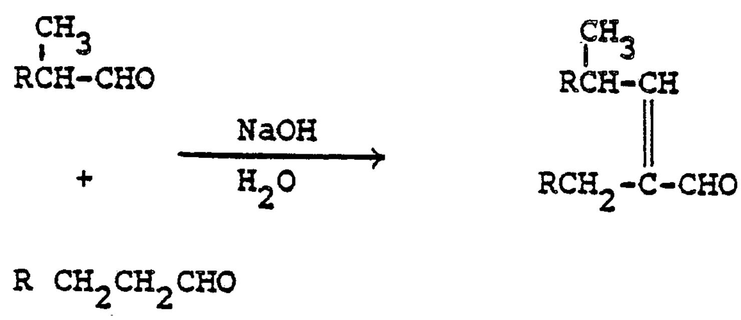


in which R represents a butyl group. However, the 2-substituted aldehydes react more slowly than other aldehydes, and therefore would constitute an undesirably high residue of unreacted component if provided to the reaction mixture in high proportion. In the present process with 75 to 80% of aldehydes without 2-branching, the aldol reaction can be conducted to include some cross-aldol of 2-substituted aldehydes so as to have yields of 85% or more based on starting aldehyde. In general, the $C_{14}$ aldehydes resulting from such cross-aldol reactions, and the alcohols produced therefrom, have properties comparable to those produced from aldol reactions of aldehydes without 2-substitution, and are suitably present in alcohol mixtures for detergent preparations as described herein. The conversion of aldehydes without 2-branching in such aldol reactions can be in the range of 95% or better while possibly only about 1/4 to 1/3 of the 2-substituted aldehydes are converted to aldol product.

In the present process the oxo product is used in the aldol reaction without any need for separation from some of the components. This contrasts with the usual commercial procedure, for example, for preparing 2-ethylhexanol as a plasticizer alcohol, wherein it is the practice to remove isobutanal before conducting an aldol reaction with n-butanal. The separation is effected by distillation and, since isomeric aldehydes have similar boiling points, separation on a commercial scale involves high capital cost equipment with consequent expense, and a substantial energy cost. There is a definite advantage in avoiding such a distillation step in the present process.

The Dimersol® dimerization process has been referred to in various publications, e.g. see "How First Dimersol is Working" by Benedek et al, Hydrocarbon Processing, May 1980, pages 143; also Chauvin et al, "The IFP Dimersol® Process for the Dimerization of $C_3$ and $C_4$ Olefinic Cuts", Advances in Petrochemical Technology, presented at American Institute of Chemical Engineers, April 13, 1976, Kansas City, Missouri.

The combination of the Dimersol® dimerization process, oxo process, aldol and reductive alkylation of amines provides a very efficient route from propylene to detergent range hydrophobes. One of the known routes to such hydrophobes relies upon oligomerization of ethylene to obtain higher molecular weight materials which are then subjected to an oxo reaction. The presently proposed route is in many respects more efficient and economical than those involving ethylene oligomerization, as propylene costs less than ethylene, and the reactions involved using dimerization, oxo and aldol are more straight forward than an oligomerization which can produce a broad mixture of products and require extensive equipment and procedures to direct it to suitable product. As discussed hereinabove, the mixture of isomers obtained from a dimerization can be carried through the oxo, aldol and amination reactions to obtain high overall conversions and yields, despite the presence of extensive branching in the materials. It is fortunate to find that a high proportion of the materials are capable of taking part sequentially in all of the required reactions, and in particular that the aldehyde failing to react to a significnant degree in the aldol reaction, because of 2-substitution, is at a comparatively low level, and it is further advantageous that the reductive alkylation is effected in good yield with high selectivity.

In the oxo stage of the present process it will be noted that cobalt catalyst is employed with the hexenes in order to promote migration of the olefinic bond and high selectivity to desired aldehyde isomers, such catalysts being for example $Co_2(CO)_8$ which may be equivalent to HCo(CO)4 under reaction conditions. The use of cobalt catalyst is particularly important for olefins with internal unsaturation.

The processes of the present invention can utilize mixtures with olefins of different carbon numbers, along with isomeric mixtures of olefins of particular carbon number. The oxo product of such mixtures can be subjected to aldol reactions as taught herein to give aldol product. Such procedures can, for example, use butenes in conjunction with Dimate® hexenes. Also, Dimate® hexenes may at times have various amounts of $C_5$, $C_7$ and $C_9$ olefins, and still be usefully employed in the present invention. Such processes will still have the advantage of production of high amounts of aldolable aldehydes in the oxo process, and result in aldol product with carbon numbers in the detergent range and with branched structure suitable for detergent use. Also, in the Dimersol® process, butene can also be dimerized or codimerized with propylene, and this provides additional ways to modify the present process by variation of the olefin feed stock, while still producing aldol product in the detergent range. In the procedures which use mixed olefins, it is also possible to conduct the oxo reaction with olefins of different carbon number, and to combine the resulting oxo products for an aldol reaction. Another olefin of particular interest for use in the present process is 4-methyl-1-pentene, which can be produced by catalyzed dimerization of propylene, as reported in Industrial Organic Chemistry, cited hereinabove. The reaction is conducted at approximately 150°C and 40 bar, using $Na/K_2CO_3$. The 4-methyl-1-pentene in an oxo process will give very high aldolable product as shown by the results for 4-methyl-pentene-2 in Example 4, and is therefore well suited for use in the present process to prepare $C_{14}$ enals and detergent derivatives thereof.

The $C_{14}$ or other detergent range alkylated amines produced by the present process can be readily converted to other detergent derivatives by known procedures. Thus quaternary ammonium salts can be prepared by heating the amines with organic chlorides, e.g. with benzyl chloride. The amine oxides can be prepared by reaction of the amines with hydrogen peroxide.

A quantity of Dimate® hexenes from a refinery stream was distilled to have a $C_6$ cut, approximately 73% of the total material. Analysis is given in Table 1. The Dimate® hexenes had been produced by dimerization of propylene over a catalyst by the Dimersol® process.

TABLE 1

Dimersol® Hexene Distribution

| | % (100% basis) | |
|---|---|---|
| 2,3-dimethyl-2-butene | 4.5 | |
| 2-methyl-2-pentene | 35.6 | |
| trans-4-methyl-2-pentene | 18.4 | |
| cis-4-methyl-2-pentene | 3.7 | |
| 2-methyl-1-pentene | 5.1 | |
| 2,3-dimethyl-1-butene+ 4-methyl-pentene-1 | 1.7 | 31.0% linear |
| trans-2-hexene | 17.8 | |
| trans-3-hexene | 6.3 | |
| cis-3 + cis-2 hexene | 6.8 | |
| 1-hexene | 0.1 | |

The distillation serves to remove some $C_5$, $C_7$ and $C_9$ hydrocarbons resulting from oligomerization involving some ethylene present in the original olefin feed, or trimerizations. It also removes $C_6$ chlorides,

along with the $C_9$ hydrocarbons; these chlorides, resulting from the dimerization catalyst, could contaminate the oxo catalyst if not removed.

It is fortunate that the hexenes mixture is amenable to reaction at a good rate in the oxo reaction. It is sufficiently reactive to permit use of moderate conditions and equipment therefor, with suitable reaction rates and times. This is in contrast to an octenes Dimate® mixture which is characterized by more relatively unreactive dibranched olefin isomers and much slower reaction rates. Such material requires more severe reaction conditions in more expensive equipment, and additional reactor capacity.

Since the oxo product is to be reacted in an aldol reaction, it is to be conducted under conditions which favor production of aldehydes as contrasted with alcohol or other products.

Example 1

Hydroformylation of a Dimate hexenes mixture, of composition reported in Table 1, was carried out in an autoclave with agitation. The autoclave was charged with 0.52 g dicobalt octacarbonyl, 74.06 Dimate hexenes and 3000 psi. gauge (20,786 KPa) of 1:1 CO and $H_2$. The autoclave was heated to 130°C and held for four hours. Liquid samples were taken every hour. The autoclave was cooled rapidly and the product removed under nitrogen. Analysis indicated 92% conversion of the olefins. The product was analyzed chromatographically, with results as reported in Table 2 (along with other examples). It will be noted that 77.9% of the aldehyde product was unbranched at the 2-position and therefore aldolable. This result was obtained, even though the hexene reactants were more than 90% composed of internal olefins, including some linear hexenes which give product little more than 50% aldolable. A similar run employing a 110°C temperature with cobalt catalyst for 23 hours is also reported.

Example 2

The autoclave of Example 1 was charged with 0.49 g of dicobalt octacarbonyl, 69.84 g 2-methylpentene-2 and 3000 psi gauge (20,786 KPa) of CO and H2 in 1 to 1 ratio. The autoclave was heated to 130°C. and held at this temperature for three hours. The autoclave was cooled and the product removed under a blanket of argon, and analyzed. Conversion of the olefin was 83%. The procedure was repeated substantially, but employing a temperature of 116°C for a 48% conversion. Results of the analysis are reported in Table 2. It is notable that better than 90% of aldehydes unbranched at the 2-position, i.e. aldolable aldehydes, were obtained. Approximately the same results were shown from samples taken during the reactions, with the 116° reaction at 1 hour giving 17% conversion and 35.4% 5-methylhexanal and 55.5% 3-methylhexanal; and the 130° reaction temperature 45% conversion at 1 hour with 32.5% 5-methylhexanal and 57.6% 3-methylhexanal.

Example 3

Hydroformylation was carried out on 2-methyl-1-pentene with cobalt catalyst in accord with the procedure of Example 2 at 116°C. for a three-hour period. A 41% conversion was obtained, with 95% of aldolable aldehyde unbranched at the 2-position being obtained. Results are reported in Table 2.

Example 4

Hydroformylation was effected with 4-methyl-2-pentene in accord with the Example 2 procedure, employing a 130°C. temperature. Results are reported in Table 2.

TABLE 2

Oxo Product Distribution

| Example | Hexene | Temp (°C) | Run Time (hours) | % I | II | - III | IV | V | VI | VII | VIII | IX | X Aldolable |
|---|---|---|---|---|---|---|---|---|---|---|---|---|---|
| 1 | D-Mixture | 130 | 23 | 13.3 | 19.4 | 0.7 | 39.1 | 4.4 | 1.1 | 1.4 | 7.3 | 1.0 | 76.9 |
| " | D-Mixture | 130 | 4 | 14.9 | 23.0 | 0.5 | 36.2 | 4.3 | 1.0 | 1.1 | 7.4 | 0.0 | 77.0 |
| " | D-Mixture | | 3 | 15.4 | 23.5 | 0.0 | 37.9 | 4.5 | 0.7 | 1.1 | 7.5 | 0.7 | 77.5 |
| " | D-Mixture | | 2 | 16.0 | 25.3 | 9.6 | 33.0 | 4.9 | 0.0 | 1.0 | 7.2 | 0.6 | 77.5 |
| " | D-Mixture | | 1 | 10.0 | 24.5 | 10.7 | 31.0 | 5.4 | trace | 1.4 | 6.0 | 0.5 | 75.1 |
| 2 | 2-methyl-2-pentene | 116 | 3 | | 34.3 | | 36.6 | | | 1.3 | 6.7 | 0.7 | 90.9 |
| " | 2-methyl-2-pentene | 130 | 3 | | 33.6 | | 36.6 | | | 1.4 | 7.2 | 1.2 | 90.2 |
| 3 | 2-methyl-1-pentene | 116 | 3 | | 10.6 | | 04.4 | | | 0.4 | 2.3 | 2.1 | 95.0 |
| 4 | 4-methyl-2-pentene | 130 | 3 | | 65.7 | | 17.3 | | trace | 1.6 | 15.3 | trace | 01.2 |
| | | | 2 | | 65.1 | | 16.6 | | | 1.6 | 16.0 | trace | 01.7 |
| | | | 1 | | 67.6 | | 14.2 | | | 1.4 | 16.0 | trace | 01.0 |

I heptanal
II 5-methylhexanal
III 3-methylhexanal
IV 3-methylhexanal
V 2-ethylpentanal

VI 3,4-dimethylpentanal
VII 2,ethyl-3-methylbutanal
VIII 2,4-dimethylpentanal
IX 2,2-dimethylpentanal

Example 5

Dimersol hexenes as described in Table 3 below were hydroformylated using a cobalt carbonyl catalyst. The cobalt was charged as a known cobalt hydroformylation catalyst precursor, cobalt acetate, .$(CH_3CO_2)_2Co.4H_2O$. and the cobalt carbonyl catalyst was formed in situ. A 967.8 g quantity of the hexenes was placed into a one gallon Magnedrive autoclave along with 65.31 g of catalyst precursor. The autoclave was purged with $H_2/CO$ (1:1 ratio) pressurized to 1650 psig (71,376 KPa gauge) and 1:1 H2/CO and heated to 130°C 3°C. The reaction pressure was then raised to 2220 psig (15,306 KPa gauge). The reaction was followed by gas uptake and the system cooled down at ~ 85% completion. Analysis of the product by GC in area % indicated the following: 15.7% hexenes, 72.4% heptanals, 3.4% heptanols and 8.5% high boiler including acetals.

The hydroformylation product was distilled and the unreacted hexenes were removed at room temperature, and 35 mm Hg. The aldehyde then distilled at 35 mm Hg from 35°C to 72°C.

Example 6

The aldol condensation of the mixed aldehydes distillate from Example 5 was conducted in a one gallon Magnedrive autoclave under an argon blanket. The co-solvent used was methanol and the volume ratio of aldehydes to catalyst solution to co-solvent was 1:1:2. The methanol co-solvent was purged with $N_2$ to remove dissolved oxygen. The methanol, 1470 ml., and the catalyst (745.1 g., 0.8M NaOH) were placed into the autoclave under argon. The autoclave was sealed and heated to 100°C at which time the 551 g (735 ml) of heptanals were added with agitation set at 100 rpm. The system was held at 100°C for 1 hour and then cooled to room temperature. The two phase product, 2502.7 g, was removed under argon. The upper phase was separated and washed with $H_2O$ which had been purged with $N_2$ to remove oxygen. The resulting upper phase tested neutral with pH paper and was vacuum stripped to remove the water and most of the unreacted heptanals. GC analysis of the upper phase before $H_2O$ wash in area%: 4.3% methanol, 14.36% heptanals, 2.07% heptanols, 77.4% tetradec-2-enals, 1.43% high boilers and 0.29% low boilers. When normalized (methanol removed), 81.27% tetradec-2-enals. After washing and vacuum stripping, GC analysis indicated at 97.71% by area was tetradec-2-enals with 2.07% heptanal and 0.22% high boilers.

Example 7

Tetradec-2-enals (50 g) from Example 6 were placed with methanol, $N_2$ purged, 50 ml, and Pd/C catalyst (2.5 g 5% Pd) in a 300 ml H.P. Magnedrive autoclave under an argon blanket. To flush the autoclave, it was sealed and pressured to 1000 psig $H_2$ (6895 KPa gauge) with hydrogen and the pressure was held with no agitation for 15 minutes; (no exotherm was observed). The $H_2$ was vented and then dimethylamine (13.6 g) was added. The hydrogen gas pressure was brought to 1000 psig (6895 KPa gauge) again and agitation started (1000 rpm). The reaction began when agitation was started as an exotherm was noted, and the reaction temperature was kept below 35°C. The reaction was complete in less than an hour and 95% of it was over in 6 minutes. Analysis by GC in an area % of the two phases: Upper phase 12.46% methanol, 2.14% N,N-dimethylheptylamines, 85.10% N,N-dimethyltetradecylamines. Lower Phase: 30.571% methanol, 2.55% dimethylamine, 1.47% N,N-dimethylheptylamines, 64.70% N,N-dimethyltetradecylamines. After distillation, 93.65% yield of the tertiary amine was obtained with the tertiary amine collected from 100° to 130°C at 5 mm of Hg. The amine was a liquid.

Example 8

The tetradec-2-enals (49.9 g) from Example 6 were first hydrogenated to the saturated aldehydes using 5% Pd/C catalyst (2.5 g) and 200 psig $H_2$ (1379 KPa gauge) at 85°C with methanol (50 ml) as solvent in a 300 ml H.P. Magnedrive autoclave. The system was cooled after 5 hours and the $H_2$ vented. The anhydrous mono-methylamine (3.5 g) was then added and hydrogen pressure (970 psig, 6687 KPa gauge) applied to the system. Heating was started but no reaction was evident till the temperature reached about 105°C. The reaction was run at 110°C under 1080 psig (7446 KPa gauge) for four hours. GC analysis confirmed by GC—MS indicated a 55% yield of the ditetradecylmethylamines with the remainder of the materials being N-methyltetrdecylamine and a small fraction of tetradecanols. The ditetradecyl-methylamine distilled at 1350°—156°, mostly 148°—150°, at $1 \times 10^{-4}$ torr, and were obtained as a liquid.

Example 9

A 300 ml stirred autoclave is charged with 43.90 g n-dodecanal which has been freshly distilled. 39.36 g methanol, 2.09 g of 5% palladium on carbon and 1000 psi gauge hydrogen. Agitation was not used until a 15 minute pressure check was achieved and the hydrogen was vented. A 11.90 g amount of dimethylamine was added and the autoclave repressurized to 1010 (6973 KPa) psig gauge. There was little reaction and the system was heated slowly to 5°C. at which temperature a noticeable reaction began. The temperature and pressure were maintained for an additional 1.5 hours after which the system was allowed to cool to 24°C and the product removed. The product was analyzed by gas-liquid chromatography and contained 73.25% N,N-dimethyl-dodecylamine, 23.27% methanol and 3.48% unknowns. While the reaction went well, it was

noted that this normal, saturated amine, dodecanal, was much less reactive than the 2-unsaturated amine utilized in Example 1 where the reaction was competed rapidly without heating.

Another sample of hexenes product from a Dimersol® dimerization refinery product was analyzed and found to have the following distribution:

TABLE 3

Hexene Distribution

| | % (100% Basis) | |
|---|---|---|
| 2,3-dimethyl-2-butene | 6.4 | |
| 2-methyl-2-pentene | 39.2 | |
| trans-4-methyl-2-pentene | 15.9 | |
| cis-4-methyl-2-pentene | 2.9 | |
| 2-methyl-1-pentene | 5.0 | |
| 2,3-dimethyl-1-butene + 4-methyl pentene-1 | 1.7 | |
| trans-2-2hexene | 16.5 | |
| trans-3-hexene | 5.8 | 28.9% |
| cis 3 + cis 2-hexene | 5.6 | |
| 1-hexene | 1.0 | |

The hexenes are suitable for conversion to detergent alcohol or amines in accord with the present invention.

Example 10

Hydroformylation was effected with 2,3-dimethyl-2-butene at 130°C, employing the procedure of Example 2. at 1, 2 and 3 hours conversions were respectively 18, 43 and 71%, and in each case the aldehyde product was analyzed as 100% 3,4-dimethylpentanal, an aldolable aldehyde.

Example 11

This example was a simulation of two oxo reactors operating at different temperatures. The autoclave was charged with 0.44 g. dicobalt octacarbonyl, and 73.94 g. Dimate hexenes. The autoclave was sealed, pressure checked and run under 3000 psig (20,786 KPa) of 1/l Co to $H_2$ at 130°C for 1 hour then heated to 140°C for an additional 4 hours. The autoclave and contents were rapidly cooled and the product removed under nitrogen. Analysis of the final product yielded that the olefin was 97% converted with 83% as the aldehydes, 11% as alcohols, and 3% high boilers. The unreacted olefin consists of 2-methylpentene-2, 2,3-dimethylbutene-2 and trans-hexene-2. The aldehydes after normalization are 39% 3-methylhexanal, 20% 5-methylhexanal, 16% heptanal, 9% 2-methylhexanal, 6% 2,4-dimethylpentanal, 5% 2-ethylpentanal, 4% 3,4-dimethylpentanal, 1% 2-ethyl-3-methylbutanal, 1% 2,2-dimethylpentanal. The aldehydes were distilled from the catalyst, unreacted starting material and other products at 20 mmHg from 41 to 45°C.

The percentage of aldehydes unbranched at the 2-position produced was 79%.

The results in Example 11 indicate that the percentage of aldehydes unbranched at the 2-position from branched hexenes (69% of hexanes) was 88.7%, compared to only 53.3% of such aldehydes from the linear hexenes (31% of hexenes). Considering only the methylpentenes (62.8%, ignoring the small amount with 2,4-dimethylbutene in Table 1), the aldolable aldehyde unbranched at the 2-position content of the aldehyde product was 86.6%).

Example 12

An aldehyde mixture representative of that from the oxo reaction of Diamate® hexenes, as described in Example 1, was reacted in an aldol reaction. An autoclave was charged with 50 ml of 0.8 M NaOH ml of 2,5-hexanediol under 20 psi (137.9 KPa) gauge argon. Then 36.2 g, 50 ml, of the aldehydes were pressured into the autoclave with argon after the autoclave had been heated to 100°C and agitation set at 1500 rpm. The reaction was run for an additional hour and the system rapidly cooled. The prduoct was removed and the

upper and lower phases were separated. The upper phase contained 16.8% unreacted aldehydes, 2.4% heptanols, 72.9% tetradecenals and 4.1% 2,5-hexanediol. The conversion of the aldehydes approximated 79%.

Example 13

This example demonstrates an aldol condensation of heptanal in glassware under conventional aldol conditions. The reaction flask was charged with 116 ml of 0.8M NaOH and the heptanal, 371.03 g, placed into the addition funnel. The system was kept under a nitrogen blanket and the agitation was set at 500 rpm. The reaction flask was heated to reflux, and the heptanal added at a constant rate such that the addition was completed after 230 minutes. A sample was removed to compare with conventional aldol results and only 33.5% of the upper phase of the sample was $C_{14}$ unsaturated aldehyde and 61% was unreacted heptanal. The system was held at reflux for an additional 10 hours after which the system was cooled. The lower phase was removed, 135.74 g, and the upper phase 336.06 g, was found to contain 66.5% 2-pentylnon-2-enal, 26.4% unreacted heptanal and 2.5% heptanol.

Example 14

This example demonstrates the efficacy of using a co-solvent with the water to enhance the aldol reaction of heptanal. The reaction flask was charged with 31 ml of 0.9M NaOH and 100 ml methanol. The addition funnel was charged with 115 g of heptanal under a nitrogen blanket. The aqueous-methanol mixture was heated to reflux with agitation of 450 rpm. The heptanal was added over a 40 minute period and refluxed for an additional 90 minutes. The system was cooled and the lower phase removed. The upper phase contains 10.1% methanol, 84.0% 2-pentylnon-2-enal, 2.0% unreacted heptanal and 1.2% heptanol. This represents 94% conversion with 98% selectivity.

Example 15

Dimerization of propylene over transition metal catalysts produces a mixture of hexenes.

The linear hexenes in the mixture can be separated and such linear hexenes are for the most part 2-hexenes. The following procedure illustrates the reaction of linear 2-hexenes in an oxo reaction, followed by an aldol reaction of the product. A sample of refinery 2-hexenes was passed through basic alumina particles for removal of oxides and 802 grams of the hexenes was placed in a 1 gallon stainless steel autoclave with 4.75 grams $Co_2(CO)_8$ catalyst. The autoclave was pressured to 2400 psi (16,547 KPa) gauge with 1:1 carbon monoxide and hydrogen, and heated to 110°C. The temperature was kept at about 110°C for 1 hour and then rose to about 130°C as the procedure was contained for about 5 hours. A 916.5 gram product was obtained, with conversion about 94% with about 78% selectivity to $C_7$ compounds, and 71.5% to $C_7$ aldehydes. Chromatography indicated the aldehydes were in ratio of about 29.4 n-heptanal to 16.7 2-methylhexanal to 8.5 2-ethylpentanal. A 907 gram amount of the product was distilled, with a final pot temperature of 120°C and vacuum of 3 mm Hg to obtain a 608 gram distillation fraction and 278 gram residue. Chromatography indicated the fraction including $C_7$ aldehydes in ratio 33.4 n-heptanal to 26.8 2-methylhexanal to 17.2 2-ethylpentanal, and minor amounts of other components. Evidently there was more loss of the normal aldehyde than the branched ones in the distillation.

It is feasible to achieve a higher percentage of n-aldehyde than present in the above distillation fraction, such as 60% or better, and therefore n-heptanal was added to the above fraction to have a more typical aldehyde for aldol reaction about 600 grams of the above fraction being used with 500 grams n-heptanal. A 300 ml amount of 0.8 molar sodium hydroxide was placed in a reaction flask with 955 ml methanol, and the aldehydes were placed in an additional funnel. The reaction medium was heated to about 71°C, and addition was slowly started and completed in about 13 hours. Chromatography indicated about 50% completion of the reaction, with $C_{14}$ aldehydes in ratio of about 23.4% 2-pentylnon-2-enal to 8.72% 2-penthyl-4-methyloct-2-enal to 1.4% 2-pentyl-4-ethylhept-2-enal. Several $C_7$ aldehydes were also present in the ratio of 22.0 heptanal to 8.5 2-methylhexanal to 5.3 2-ethylpentanal.

The aldol condensation product was hydrogenated over a cobalt on Kieselguhr catalyst, using 131 grams catalyst with 1336 grams of the condensation product. The materials were maintained at about 160°C and 1500 psi gauge (10,645 KPa) of hydrogen for about two hours when reaction appeared complete. Reaction conditions were maintained for an additional 4.5 hours. Analysis indicated about 99% completion of the hydrogenation. The product contained 2-pentyl-nonanol in about 18.4 to 7.2 ratio to a mixture of 2-pentyl-5-methyl-octanol and 2-pentyl-4-ethyl-heptanol and large amounts of $C_7$ alcohols from the unreacted aldehyde, being heptanol in a 27.5 to 16.3 ratio to a mixture of 2-methyl-hexanol and 2-ethylpentanol. The product was fractionated by distillation, with a 280 gram fraction being obtained at 110—115°C at 2 mm Hg from 1180 grams of hydrogenation product. The fraction was in large predominance composed of $C_{14}$ alcohols being obtained at 110—115°C at 2 mm Hg from 1180 grams of hydrogenation product. The fraction was in large predominance composed of $C_{14}$ alcohols.

Example 16

A mixture of hexenes produced by the Dimersol® dimerization process was utilized as olefin reactant. The crude hexene cut from the dimerization was used, and had the distribution of linear and branched hexenes typical of such material. A 1029 gram amount of the hexenes was used in a 1 gallon autoclave with

6.04 grams catalyst, $Co_2(CO)_8$, 0.02 weight %. Peroxides had been removed from the hexenes by treatment on a basic alumina column. The autoclave was taken to reaction conditions with 1:1 $CO/H_2$ and maintained at 110°C and 2600 psi gauge (18028 KPa) for 9 hours, with 80% of theoretical gas uptake, and then continued overnight. Chromatography indicated high conversion to $C_7$ aldehydes, with minor amounts of residual hexenes. A 1360 gram amount of the product was subjected to distillation, with a 797 gram fraction being obtained at pot temperature of 60 to 97°C as the vacuum dropped from 90 mm Hg to 5 mm Hg. Chromatography indicated a high portion of $C_7$ aldehydes with a very small amount of $C_6$ olefins.

A 792 gram amount of the above aldehyde fraction was utilized in an aldol reaction, adding the aldehyde material from an additional funnel to a reaction flask containing 564 grams methanol and 250.9 grams 0.8 molar sodium hydroxide. The addition took 6 hours, with stirring at about 500 rpm and temperature at 72—73°C. The reaction mixture was then refluxed for 1.5 hours. Analysis of a sample indicated only about 1 part aldol product to 3 parts aldehyde, on a mole basis. The reaction was continued at reflux overnight, giving 1 part aldol product to about 2.6 parts aldehyde reactant. During the reaction it was observed that the reaction mixture had a large upper phase and a smaller lower phase, indicating that methanol was not very effective in promoting miscibility and reaction, possibly because of the relatively long chain length of $C_7$ aldehydes. Chromatography showed a fair amount of the $C_{14}$ aldol product, including 2-pentylnonenal, and a large amount of unreacted $C_7$ aldehydes. (Results were better in Example 10 above in which a higher proportion of methanol was employed).

The above aldol product was subjected to further aldol reaction, after removing the methanol to employ condensate, 55.6 area percent $C_7$ aldehydes and 31.5 area percent $C_{14}$ enals, was placed in an addition funnel and added to a reaction flask containing 163 grams 0.8M NaOH and 389 grams 2,5-hexanediol. Addition was completed after 45 minutes, with temperature maintained at 100°C with agitation of the reaction mixture. The reaction mixture was then refluxed at 100°C for 1.75 hours. The reaction mixture separated into upper and lower phases of about equal weight. The conversion had been improved in that the ratio of $C_{14}$ enals to $C_7$ aldehydes in the product (upper phase) was now about 1.7 to 1. A 515 gram amount of the product was subjected to hydrogenation, employing 51.65 grams cobalt on Kieselguhr catalyst and 160°C, about 1580 psi gauge (10,995 KPa) hydrogen. Approximately 549 grams of product was recovered. The conversion of $C_{14}$ saturated alcohols was about 90%, with about 10% found as unsaturated alcohols. The product was filtered to remove catalyst, and the filtrate was distilled. The process produced several $C_{14}$ alcohols in very substantial amounts, with a number of others in very small amounts. Several $C_7$ alcohols from unreacted aldehyde were also present in substantial amount.

### Example 17

A freshly distilled sample of 2-pentene was hydroformylated in a 300 ml autoclave, employing 0.41 gram $Co(CO)_8$ catalyst with 65.84 grams pentene. A 1:1 mixture of $CO/H_2$ was used, with initial charge to 1500 psi (10,342 KPa) gauge and heating to 120°C and 3000 psi (20,684 KPa) with agitation at 1000 rpm. Gas-uptake was observed, as the pressure was increased to 3000 psi (20,684 KPa). After about 2 hours, an 83 gram product was obtained. Chromatography showed a small residual amount of pentene and $C_6$ aldehydes in the ratio of 61.1 hexanal to 28.9 2-methylpentanal to 10.0 2-ethylbutanal. An aldehyde sample was provided to have aldehydes in the same ratio, using 189.1 grams addition to 110 ml of 0.8M NaOH in a round bottom flask equipped with a mechanical stirrer. Heating was begun and addition was started after about 15 minutes and continued as reflux started around 91°C. Addition was completed in about 40 minutes. Stirring was continued for an hour, but without further heating, and a sample was taken. Analysis indicated partial reaction. The reaction mixture was heated to 95°C for an additional 1½ hours. Upper and lower phases of the reaction mixture were separated, and the upper phase was analyzed. The analysis indicated better than 25% conversion to $C_{12}$ enal, nearly all being 2-butyloctenal, and large amounts of unreacted $C_6$ aldehydes, the major parts of which was branched aldehydes. The aldehyde mixture can readily be hydrogenated to the corresponding alcohols or aminated to alkyl substituted amines.

### Example 18

An aldol procedure was carried out as in Example 17 except that the amount of water was increased ten fold. The same amount of NaOH was present, although now in much more dilute solution. Because of the large volume, less effective stirring was achieved. After a two hour reaction, analysis indicated substantial conversion to $C_{12}$ enals, although somewhat lower than in Example 16.

### Example 19

An aldol reaction was carried out as in Example 17, employing 6.1 to 2.9 to 1 ratio of hexanal to 2-methylpentanal to 2-ethylbutanal, the total amount being 310 grams. The branched aldehydes were placed in a flask with 110 ml of 0.8M NaOH, and tetrabutylammonium chloride in an amount molecularly equivalent to the NaOH. The tetrabutyl-ammonium chloride serves as a phase transfer catalyst. The reaction flask was heated to reflux and addition was started. The addition continued for about 6 hours with reflux temperatures (pot) from 90—95°C. The mixture was cooled and separated into two phases. Analysis of the upper phase showed better than 75% conversion to $C_{12}$ enals, about half of which was 2-butyloctenal, and the remainder mainly a mixture of 2-butyl-4-ethyl-heptanal and 2-butyl-4-ethylhexenal. In the unreacted $C_6$ aldehydes present, the branched aldehydes were in greater amount the hexanal. The

reaction can be directed to produce a higher percentage of product from the n-hexanal by adding the aldehydes together, rather than adding the n-aldehyde to the branched aldehydes in the reaction mixture as in the foregoing procedures. The phase transfer catalyst was effective in improving conversion in this procedure, but use of co-solvents, such as methanol or diols, may be more practical for large scale continuous operations. The use of hexanediol has been shown effective for aldol reaction of heptanals herein, and can similarly be used with hexanals. It will be noted that the alcohols and amines produced from both the pentenes and hexenes feedstocks are intended for use as detergent range alcohols and amines. The consideration herein as to reaction conditions and various parameters of the oxo and aldol reactions as described for the hexenes and resulting $C_7$ aldehydes also are in general applicable to the pentenes and resulting $C_6$ aldehydes. The $C_{12}$ alcohols and amines produced from the reactions starting with pentenes will have the hydrophobic groups such compounds provide in detergents, and the groups will have a degree of branching similar to that of $C_{14}$ compounds from hexenes. It is feasible to substantially avoid presence of branches on adjacent carbon atoms. In one particular aspect the present invention is directed to a process of preparing amines from an oxo reaction with olefins selected from those having 5 to 6 carbon atoms, or mixtures thereof, to obtain aldehydes having 6 to 7 carbon atoms, comprising high amounts of aldehydes without 2-substitution and effecting aldol conversion with limited participation of 2-substituted aldehyde to obtain aldol product, which is then aminated to $C_{12}$ or $C_{14}$ amines having properties valuable for use in detergents.

### Example 20

An aldol reaction was conducted in a 1 liter round bottom flask equipped with stirrer, additional funnel, reflux condenser and adaptors for nitrogen flow. A 100 ml amount of aqueous 1 molar potassium hydroxide solution was placed in the flask and heated to about 85°C n-Pentanal and 2-methylbutanal were admixed in about 3:1 ratio, after each had been purified by distillation, and used for gradual addition to the reaction flask with stirring. Over about one hour, about 300 ml was added containing 187.7 grams n-pentanal and 60.8 grams 2-methylbutanal. The reaction mixture was placed in a separatory funnel; and the lower aqueous phase (87 grams) was separated. The organic layer was washed four times with water and amounted to 213.3 grams. Gas chromatography analysis for the starting aldehydes indicated about a 3:1 ratio of 2-methylbutanal to n-pentanal, showing that the n-pentanal had been consumed at a much higher rate in the reaction. The product contained about 70.5% of alkenal condensation product and 25.3% of the starting aldehydes. The product had 2-propyl-heptanal in about 9:1 ratio to 2-propyl-4-methyl-hexanal.

A 110.89 gram amount of the product was utilized for hydrogenation, employing 11 grams of cobalt on Kieselguhr catalyst with 4.4 ml $H_2O$ as promoter, in a 300 ml stirred autoclave. The autoclave was pressured to 1000 psi (6895 KPa) with hydrogen and gradually heated, with hydrogen uptake starting at about 40°C. After one hour, the pressure had fallen to 480 psi, and the autoclave was again pressured to 1000 psi (6895 KPa). After two hours, with further addition of hydrogen, the pressure was 1510 psi (10,414 KPa) and temperature 160°C. The run was continued for a total of sixteen hours. The measured gas uptake was in very slight excess of theory for hydrogenation of both the olefin and aldehyde groups in the compounds present.

The product was filtered through a Celite filter mat to remove catalyst, and the mat was washed with n-hexane. The n-hexane was removed under vacuum, leaving 88 grams of product for distillation. Distillation was carried out at 10 mm Hg, with 18.1 gram being collected at 30—100°C, which gas chromatography indicated to be 66.4% 2-methylbutanol, 27.5% pentanol, and 4.1% 2-propylheptanol. An additional 38.9 grams was collected at 103.5—105°C, 11.3% 2-propyl-4-methylhexanol and 87.7% 2-propylheptanol. It can be seen that the above described procedure provides 2-propylheptanol with only very minor adulteration by the aldol alcohols product of branched aldehydes. Also the 2-propylheptanol from the 2-methylbutanol produced by hydrogenation of the 2-methylbutanal which did not undergo the aldol condensation.

### Example 21

An aldehyde mixture representative of that from the oxo reaction of Dimate® hexenes, as described in Example 1, was reacted in an aldol condensation. A stirred autoclave was charged with 735 ml 0.8M NaOH, 1470 ml methane and 20 psig (137.9 KPa gauge) argon (KPa). The autoclave was heated to 100°C and 735 ml (551.0 g) of the aldehydes were pressurized into the autoclave with argon. The reaction was run for an additional hour and the system was rapidly cooled. The product was removed and the upper and lower phases were separated. The upper phase contained 14.4% unreacted aldehydes, 2.1% heptanols, 4.4% methanol, 1.6% high boilers and 77.5% tetradecenals. The tetradecenals product consists of some 28 isomers in amounts as named and illustrated in Table 4 where the aldehydic function is designated by an asterisk. The aldehydes are suitable for alkylation of amines in accord with the present invention.

TABLE 4

| Structure | Compound name | Weight % | Aldol Condensation Substrates<br>Carbanion | <br>Acceptor |
|---|---|---|---|---|
| C-C-C-C-C-C=C-C-C-C-C<br>C C* C | (E+Z)-2-(3-Methylbutyl)-5-Methyl-2-Octenal | 25.4 | 5-Methylhexenal | 3-Methylhexenal |
| C<br>C-C-C-C-C-C=C-C-C-C-C<br>C C* | (+Z)-2-(1-Methylbutyl)-5-Methyl-2-Octenal | 14.8 | 3-Methylhexanal | 3-Methylhexanal |
| C-C-C-C-C-C-=C-C-C-C-C-C<br>C C* | (+Z)-2-Pentyl-5-Methyl-2-Octenal | 13.4 | Heptanal | 3-Methylhexanal |
| C-C-C-C-C-C=C-C-C-C-C<br>C C* C | (E+Z)-2-(3-Methylbutyl)-7-Methyl-2-Octenal | 7.0 | 5-Methylhexanal | 5-Methylhexanal |
| C-C-C-C-C-C-C=C-C-C-C-C-C<br>C* | (E*Z)-2-Pentyl-2-Nonenal | 6.0 | Heptanal | Heptanal |
| C<br>C-C-C-C-C=C-C-C-C-C<br>C C* C | (E*Z)-2-(3-Methylbutyl)-5,6-Dimethyl-2-Heptenal | + | 5-Methylhexenal | 3,4-Dimethylhexanal |
| C C<br>C-C-C-C-C=C-C-C-C-C<br>C C* | (E+Z)-2-(1-Methylbutyl)-5,6-Dimethyl-2-Heptenal | 5.1 | 3-Methylhexanal | 3,4-Dimethylpentanal |
| C-C-C-C-C-C=C-C-C-C-C-C<br>C C* | (E*Z)-2-Pentyl-7-Methyl-2-Octenal | 4.1 | Heptanal | 5-Methylhexanal |
| C-C-C-C-C-C-C=C-C-C-C-C<br>C* C | (E*Z)-2-(3-Methylbutyl)-2-Nonenal | 4.1 | 5-Methylhexanal | Heptanal |
| C<br>C-C-C-C-C-C=C-C-C-C-C<br>C C* | (E*Z)-2-(1-Methylbutyl)-7-Methyl-2-Octenal | 2.4 | 3-Methylhexanal | 5-Methylhexanal |
| C-C-C-C-C-C=C-C-C-C-C-C<br>C C* | (E+Z)-2-Pentyl-4-Methyl-2-Octenal | 2.4 | Heptanal | 2-Methylhexanal |
| C<br>C-C-C-C-C=C-C-C-C-C-C<br>C C* | (E+Z)-2-Pentyl-5,6-Dimethyl-2-Heptenal | 2.3 | Heptanal | 3,4-Dimethylpentanal |
| C-C-C-C-C-C=C-C-C-C-C<br>C C* C | (E+Z)-2-(3-Methylbutyl)-4-Methyl-2-Octenal | 2.2 | 5-Methylhexanal | 2-Methylhexanal |
| C<br>C-C-C-C-C-C-C=C-C-C-C-C<br>C* | (E+Z)-2-(1-Methylbutyl)-2-Nonenal | 1.5 | 3-Methylhexanal | Heptanal |
| C<br>C-C-C-C-C=C-C-C-C-C<br>C C C* | (E+Z)-2-(1-Methylbutyl)-4-Ethyl-2-Heptenal | 0.7 | 3-Methylhexanal | 2-Ethylpentanal |

TABLE 4 continued

| Structure | Compound name | Weight % | Aldol Condensation Substrates Carbanion | Acceptor |
|---|---|---|---|---|
| C-C-C-C-C=C-C-C-C-C<br>| C*<br>C-C | (E*Z)-2-Pentyl-4-Ethyl-2-Heptenal | 0.6 | Heptanal | 2-Ethylpentanal |
| C<br>C-C-C-C-C-C-C=C-C-C-C-<br>C* C | (E+Z)-2-(1,2-Dimethylpropyl)-2-Nonenal | 0.5 | 3,4-Dimethylpentanal | Heptanal |
| C<br>C-C-C-C-C-C=C-C-C-C-C<br>C C* | (E+Z)-2-(1-Methylbutyl)-4-Methyl-2-Octenal | 0.4 | 3-Methylhexanal | 2-Methylhexanal |
| C-C-C-C-C=C-C-C-C<br>C C C* C | (E+Z)-2-(3-Methylbutyl)-4-Ethyl-2-Heptenal | 0.2 | 5-Methylhexanal | 2-Ethylpentanal |
| C C<br>C-C-C-C-C=C-C-C-C<br>C C* C | (E+Z)-2-(1,2-Dimethylpropyl)-5,6-Dimethyl-2-Heptenal | UNK | 3,4-Dimethylpentanal | 3,4-Dimethylpentanal |

Example 22

This experiment and Example 23 illustrates the utility of the invention for mixtures of olefins. Hydroformylation of Dimate® hexenes and butenes was carried out in an autoclave with agitation. The autoclave was charged with 2.61 g of dicobalt octacarbonyl (catalyst precursor), 270.2 g of Dimate® hexenes and 180.9 g butenes and 3000 psi gauge (20.786 KPa) of 1:1 CO and $H_2$. The autoclave was heated to 120°C and held for two hours. The autoclave and contents were cooled rapidly and the product removed under argon. The product was analyzed chromatographically as reported in Table 5.

TABLE 5

| Dimate® hexene, butene-2 Hydroformylation Products Compound | Area % |
|---|---|
| Butene-2 | 1.5 |
| Dimate hexenes | 9.6 |
| 2,2-dimethylpentanal | 0.5 |
| 2,4-dimethylpentanal | 2.1 |
| 2-methylbutanal | 10.0 |
| Pentanal | 23.6 |
| 2-ethyl-3-methylbutanal | 0.4 |
| 2-ethylpentanal | 3.3 |
| 3,4-dimethylpenntanal | 0.5 |
| 2-methylhexanal | 3.2 |
| 3-methylhexanal | 18.5 |
| 5-methylhexanal | 12.5 |
| Heptanal | 9.3 |
| High Boilers (including heptyl alcohols and Formates) | 5.0 |

The percent of aldehyde production without 2-substitution was over 76%.

Example 23

An aldehyde mixture representative of that from the oxo reaction of Dimate® hexenes and butene as described in Example 22 was reacted in an aldol condensation. A stirred autoclave was charged with 313 ml methanol, 313 ml 0.8M NaOH, and 110 psi (758.4 KPa) gauge argon. The system was heated to 100°C and the mixture of aldehydes, 313 ml, was pressured into the autoclave with argon. The reaction was run an additional one hour and thirty minutes and the system rapidly cooled. The product was removed and the upper phase and lower phases were separated. The upper phase contained 9.4% unreacted pentanals, 20.6% unreacted heptanals, 26.9% decenals, 27.1% 2-dodecenals, 6.3% 2-tetradecenals, and 0.8% high boilers. The skeletel structures of the 2-decenals and the 2-dodecenals are given in TABLE 6. The enals can readily be converted to alkylated amines in accord with procedures described herein.

TABLE 6

| Structure | Compound Name |
| --- | --- |
| C-C-C-C-C=C-C-C-C<br>C* | (E+Z)-2-Propyl-2-Heptenal |
| | 2-Dodecenals |
| C-C-C-C-C-C-C=C-C-C-C<br>C* | (E+Z)-2-Propyl-2-Nonenal |
| C-C-C-C-C-C=C-C-C-C<br>C C* | (E+Z)-2-Propyl-7-Methyl-2-Octenal |
| C-C-C-C-C-C=C-C-C-C<br>C C* | (E+Z)-2-Propyl-5-Methyl-2-Octenal |
| C<br>C-C-C-C-C=C-C-C-C-C<br>C* | (E+Z)-2-(1-Methylbutyl)-2-Heptenal |
| C-C-C-C-C=C-C-C-C-C<br>C* C | (E–Z)-2-(3-Methylbutyl)-3-Heptenal |
| C-C-C-C-C=C-C-C-C-C-C<br>C* | (E+Z)-2-Pentyl-2-Heptenal |
| C<br>C-C-C-C-C=C-C-C-C<br>C* C | (E+Z)-2-(1,2-Dimethylpropyl)-2-Heptenal |
| C<br>C-C-C-C-C=C-C-C-C<br>C C* | (E+Z)-2-Propyl-4,5-Dimethyl-2-Heptenal |
| C-C-C-C-C-C=C-C-C-C<br>C C* | (E+Z)-2-Propyl-4-Methyl-2-Octenal |
| C-C-C-C-C=C-C-C-C<br>| C*<br>C-C | (E+Z)-2-Propyl-4-Ethyl-2-Heptenal |

The structure of most of the product is represented by the followng formula:

Structure of $C_{10}$ to $C_{14}$ Enals from Aldol of Pentanal with heptanals from Oxo of Dimate Hexenes.

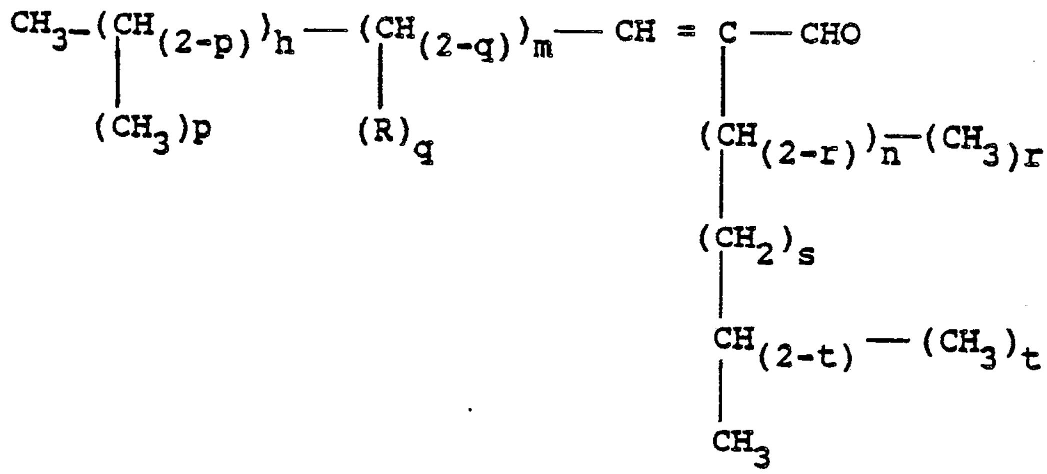

Wherein:

R = methyl or ethyl; h and m = 0, 1, 2, 3, 4 and 5;
n and s = 0, 1, 2; p, q, r and t = 0 or 1;
m + h can never = more than 5;
q and p = 0 when m + h = 5 or when m + h = 3;
h = 3 and m = 1 when R = methyl and q = 1 and p = 0;
q = 0 and p = 1 when h = 2 and m = 2 or when h = 1 and m = 3;
n + s can never = more than 3;
r and t = 0 when n + s = 3 or when n + s = 1;
s = 0 when n, r and t = 1 or when n = 2 and r = 0 and t = 1;
s = 1 when n = 1 and t and r = 0 or 1 but only one of t and r can be 1;
s = 2 when t = 1 and n = 0;
h = 2 and m = 1 when R = ethyl and q = 1 and p = 0.

Example 24

The autoclave of example 1 was charged with 0.25 g carbonylbis (triphenylphosphine) rhodium (I) chloride, 11.78 g triphenylphosphine, 46.20 g 3-methylbutene-1 and 300 psi (2068.4 KPa) gauge 1:1 CO and $H_2$. The autoclave was heated to 130°C and held at this temperature for one hour and 35 minutes. The autoclave was cooled and the product removed under a blanket of argon and analyzed. The product is 83% 4-methylpentanal with 7% 3-methylpentanal and 7% unreacted 3-methylbutene-1 starting material.

Example 25

Hydroformylation was carried out on 2-methylbutene-1 with rhodium catalyst in accord with the procedure of Example 20 at 100°C for an eight hour fifteen minute period. A 49% conversion was obtained with 98% selectivity to 3-methylpentanal.

Example 26

An aldehyde mixture representative of that from the oxo reactions as described above n Examples 24 and 25 was reacted in an aldol condensation. An autoclave was charged with 50 ml 0.8M NaOH, 50 ml methanol, and 10 psig (68.9 KPa) argon. The autoclave was heated to 100°C with agitation set at 1500 rpm and the mixture of 23.08 g 3-methylpentanal and 23.11 g 4-methylpentanal, 50 ml total, were pressured into the autoclave with argon. The reaction was run for an additional hour and the system rapidly cooled. The product was removed and the upper and lower phases methanol were separated. The upper phase contained 2% methanol, 4.0% unreacted 3-methylpentanal, 2.6% unreacted 4-methylpentanal, 0.4% hexanols, 0.6% high boilers with 92.4% as dodecenals. The skeletal structure of the dodecenals are the same as the first four structures of dodecenals listed in Table 7 which details skeletal structures of a mixed pentene stream with Dimate® hexenes, if hydroformylated with cobalt catalyst and aldol condensed as in Examples 21 and 22.

TABLE 7

| Structure | Compound Name |
|---|---|
| C-C-C-C-C=C-C-C-C<br>C C* C | (E+Z)-2-(2-Methylpropyl)-6-Methyl-2-Heptenal |
| C-C-C-C-C=C-C-C-C<br>C C* C | (E+Z)-2-(2-Methylpropyl)-5-Methyl-2-Heptenal |
| C<br>C-C-C-C-C=C-C-C-C<br>C C* | (E+Z)-2-(1-Methylpropyl)-6-Methyl-2-Heptenal |
| C<br>C-C-C-C-C=C-C-C-C-<br>C C* | (E+Z)-2-(1-Methylpropyl)-5-Methyl-2-Heptenal |
| C-C-C-C-C-C=C-C-C-C-C<br>C* | (E+Z)-2-Butyl-2-Octenal |
| C-C-C-C-C=C-C-C-C-C<br>C C* | (E+Z)-2-Butyl-6-Methyl-2-Heptenal |
| C-C-C-C-C=C-C-C-C-C<br>C C* | (E+Z)-2-Butyl-5-Methyl-2-Heptenal |
| C<br>C-C-C-C=C-C-C-C-C<br>C C* | (E+Z)-2-Butyl-4,5-Dimethyl-2-Hexenal |
| C<br>C-C-C-C-C-C=C-C-C-C<br>C* | (E+Z)-2-(1-Methylpropyl)-2-Octenal |
| C-C-C-C-C-C=C-C-C-C<br>C* C | (E+Z)-2-(2-Methylpropyl)-2-Octenal |
| C C<br>C-C-C-C=C-C-C-C<br>C C* | (E+Z)-2-(1-Methylpropyl)-4,5-Dimethyl-2-Hexenal |
| C-C-C-C-C=C-C-C-C-C<br>C C* | (E+Z)-2-Butyl-4-Methyl-2-Heptenal |
| C-C-C-C=C-C-C-C-C<br>\| C*<br>C-C | (E+Z)-2-Butyl-2-Ethyl-2-Hexenal |
| C<br>C-C-C-C=C-C-C-C-C-<br>C C | (E+Z)-2-Butyl-4,5-Dimethyl-2-Hexenal |
| C-C-C-C-C-C-C=C-C-C-C-C<br>C* | (E+Z)-2-Butyl-2-Nonenal |
| C-C-C-C-C=C-C-C-C-C-C<br>C* | (E+Z)-2-Pentyl-2-Heptenal |
| C-C-C-C-C-C-C=C-C-C-C-<br>C* C | (E+Z)-2-(2-Methylpropyl)-2-Nonenal |

TABLE 7 continued

| Structure | Compound Name |
|---|---|
| C-C-C-C-C=C-C-C-C-C-C<br>C C* | (E+Z)-2-Pentyl-4-Methyl-2-Heptenal |
| C-C-C-C-C=C-C-C-C-C-C<br>C C* | (E+Z)-2-Pentyl-5-Methyl-2-Heptenal |
| C-C-C-C-C=C-C-C-C-C-C<br>C C* | (E+Z)-2-Pentyl-6-Methyl-2-Heptenal |
| C-C-C-C-C-C=C-C-C-C-C<br>C C* | (E+Z)-2-Butyl-7-Methyl-2-Octenal |
| C<br>C-C-C-C-C=C-C-C-C-C<br>C C* | (E+Z)-2-Butyl-5,6-Dimethyl-2-Heptenal |
| C<br>C-C-C-C-=C-C-C-C-C-C<br>C C* | (E+Z)-2-Pentyl-4,5-Dimethyl-2-Hexenal |
| C<br>C-C-C-C-C-C-C=C-C-C-C-<br>C* | (E+Z)-2-(1-Methylpropyl)-2-Nonenal |
| C<br>C-C-C-C-C-C=C-C-C-C<br>C* C | (E+Z)-2-(1,2-Dimethylpropyl)-2-Octenal |
| C-C-C-C-C=C-C-C-C-C<br>\| C*<br>C-C | (E+Z)-2-Butyl-4-Ethyl-2-Heptenal |
| C-C-C-C=C-C-C-C-C-C<br>\| C*<br>C-C | (E+Z)-2-Pentyl-4-Ethyl-2-Hexenal |
| C-C-C-C-C=C-C-C-C<br>C C* C | (E+Z)-2-(2-Methylpropyl)-6-Methyl-2-Heptenal |
| C<br>C-C-C-C=C-C-C-C<br>C C* C | (E+Z)-2-(2-Methylpropyl)-4,5-Dimethyl-2-Hexenal |
| C-C-C-C-C-C=C-C-C-C<br>C C* C | (E+Z)-2-(2-Methylpropyl)-5-Methyl-2-Octenal |
| C<br>C-C-C-C-C=C-C-C-C-C<br>C C* | (E+Z)-2-(1-Methylbutyl)-5-Methyl-2-Heptenal |
| C-C-C-C-C=C-C-C-C-C<br>C C* C | (E+Z)-2-(3-Methylbutyl)-6-Methyl-2-Heptenal |
| C<br>C-C-C-C-C=C-C-C-C-C<br>C C* C | (E+Z)-2-(3-Methylbutyl)-4,5-Dimethyl-2-Heptenal |

TABLE 7 continued

| Structure | Compound Name |
|---|---|
| C C<br>C-C-C-C-C=C-C-C-C<br>C C* | (E+Z)-2-(1-Methylpropyl)-4,5-Dimethyl-2-Heptenal |
| C<br>C-C-C-C-C-C=C-C-C-C-<br>C C* | (E+Z)-2-(1-Methylpropyl)-7-Methyl-2-Octenal |
| C<br>C-C-C-C-C=C-C-C-C-C<br>C C* | (E+Z)-2-(1-Methylbutyl)-6-Methyl-2-Heptenal |
| C-C-C-C-C=C-C-C-C-C<br>C C* C | (E+Z)-2-(3-Methylbutyl)-4-Methyl-2-Heptenal |
| C<br>C-C-C-C=C-C-C-C-C<br>\| C*<br>C-C | (E+Z)-2-(1-Methylbutyl)-4-Ethyl-2-Hexenal |
| C<br>C-C-C-C-C=C-C-C-C<br>\| C*<br>C-C | (E+Z)-2-(1-Methylpropyl)-4-Ethyl-2-Heptenal |
| C<br>C-C-C-C-C=C-C-C-C-C<br>C C* | (E+Z)-2-(1-Methylbutyl)-4-Methyl-2-Heptenal |
| C-C-C-C=C-C-C-C-C<br>\| C* C<br>C-C | (E+Z)-2-(3-Methylbutyl)-4-Ethyl-2-Hexenal |
| C-C-C-C-(=C-C-C-C<br>\| C* C<br>C-C | (E+Z)-2-(2-Methylpropyl)-4-Ethyl-2-Heptenal |
| C<br>C-C-C-C-C=C-C-C-C<br>C C* C | (E+Z)-2-(1,2-Dimethylpropyl)-6-Methyl-2-Heptenal |
| C<br>C-C-C-C=C-C-C<br>C C* C | (E+Z)-2-(1,2-Dimethylpropyl)-5-Methyl-2-Hexenal |
| C<br>C-C-C-C-C=C-C-C-C<br>C C* C | (E+Z)-2-(1,2-Dimethylpropyl)-4-Methyl-2-Heptenal |
| C<br>C-C-C-C=C-C-C-C<br>\| C* C<br>C-C | (E+Z)-2-(1,2-Dimethylpropyl)-4-Ethyl-2-Hexenal |
| C<br>C-C-C-C-C-C=C-C-C-C<br>C C* | (E−Z)-2-(2-Methylpropyl)-6-Methyl-2-Octenal |
| C<br>C-C-C-C-C=C-C-C-C-C<br>C C* | (E−Z)-2-(1-Methylbutyl)-4-Methyl-2-Heptenal |

TABLE 7 continued

| Structure | Compound Name |
|---|---|
| C<br>C-C-C-C-C=C-C-C-C<br>C C* C | (E–Z)-2-(1,2-Dimethylpropyl)-5-Methyl-2-Heptenal |
| C-C-C-C-C-C=C-C-C-C-C<br>C* C | (E–Z)-2-(3-Methylbutyl)-2-Octenal |
| C-C-C-C-C-C=C-C-C-C-C<br>C C* | (E–Z)-2-Butyl-5-Methyl-2-Octenal |
| C<br>C-C-C-C-C-C=C-C-C-C-C<br>C* | (E–Z)-2-(1-Methylbutyl)-2-Octenal |
| C-C-C-C-C=C-C-C-C-C<br>C C* C | (E–Z)-2-(3-Methylbutyl)-5-Methyl-Heptenal |
| C<br>C-C-C-C=C-C-C-C<br>C C* C | (E–Z)-2-(1,2-Dimethylpropyl)-6-Methyl-2-Heptenal |
| C C<br>C-C-C-C=C-C-C-C-C<br>C C* | (E–Z)-2-(1-Methylbutyl)-4,5-Dimethyl-2-Hexenal |
| C C<br>C-C-C-C=C-C-C-C<br>C C* C | (E–Z)-2-(1,2-Dimethylpropyl)-4,5-Dimethyl-2-Hexenal |
| C<br>C-C-C-C-C-C=C-C-C-C<br>C C* | (E–Z)-2-(2-Methylpropyl)-4-Methyl-2-Octenal |
| C<br>C-C-C-C-C-C=C-C-C-C<br>C C* | (E–Z)-2-(1-Methylpropyl)-4-Methyl-2-Octenal |
| C-C-C-C-C-C=C-C-C-C-C<br>C C* | (E–Z)-2-Butyl-4-Methyl-2-Octenal |
| C<br>C-C-C-C=C-C-C-C<br>\| C* C<br>C-C | (E–Z)-2-(1,2-Dimethylpropyl)-4-Ethyl-2-Octenal |
| C-C-C-C-C-C=C-C-C-C<br>C* C | (E–Z)-2-(2-Methylpropyl)-2-Octenal |
| C<br>C-C-C-C=C-C-C-C-C<br>C C* | (E–Z)-2-Butyl-4,5-Dimethyl-2-Hexenal |
| C<br>C-C-C-C-C-C=C-C-C-C-<br>C* | (E–Z)-2-(1-Methylpropyl)-2-Octenal |

Similarly, such oxo-aldol reactions of a predominant component of isoamylene streams, 2-methylbutene-2, would produce an enal mixture with most components represented by the structure:

Structures of Dodec-4-enals from Oxo-Aldols of 2-Methylbutene-2

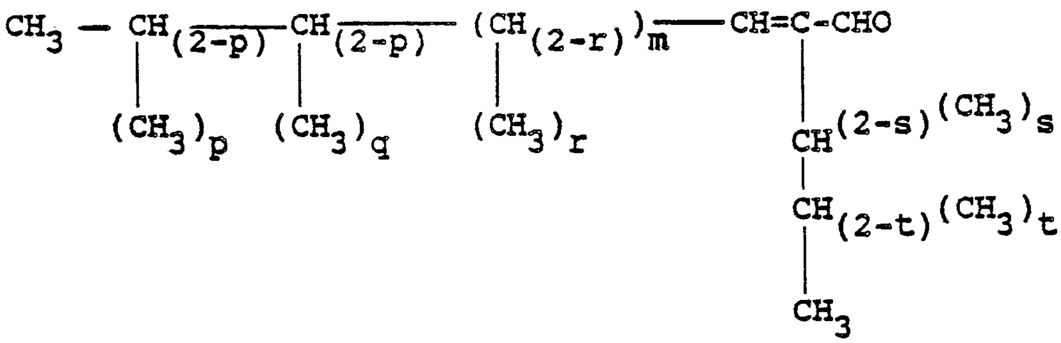

Wherein:

m = 0 or 1;
p, q, r, s, t = 0 or 1 but only one of s and t can be 1;
m = 1 when r = o and only one of p and q can be 1;
m = 0 when p and q = 1

At the oxo stage of the reaction the main aldehydes produced are 3-methylpentanal-1 and 4-methylpentanal-1 with possibly up to 10% 2,3-dimethylbutanal-1. The aldol products are mainly 5-methyl-2-(1-methylpropyl)hept-2-enal-1, 6-methyl-2-(methylpropyl)-hept-2-enal-1, 6-methyl-2-(1-methylpropyl)-hept-2-enal-1 and 5-methyl-2-(2-methylpropyl)hept-2-enal-1, and two C10 aldehydes in very small amounts.

## Example 27

This example illustrates the utility of the invention for synthesis of $C_{11}$ and $C_{14}$ unsaturated aldehydes.

An aldehyde mixture representive of that from the oxo reaction of Dimate® hexenes, as described above in Example 1 was reacted in an aldol condensation along with normal butanal. A stirred autoclave was charged with 600 ml 0.8M NaOH, 1160 ml methanol and 20 psig (137.8 KPa) argon. The autoclave was heated to 100°C and a mixture of 323.2 g heptanals from oxo of Dimate® hexenes and 212.9 g butanal, total volume 600 ml was pressured into the autoclave with argon. The reaction was run for an additional hour and the system rapidly cooled. The product was removed and the upper and lower phases were separated. The upper phase contained 2.7% methanol, 5.1% unreacted heptanals, 13.7% (Z+E)-2-ethyl-hexenal, 56.0% 2-undecenals, 18.4% 2-tetradecenals, 0.5% heptanols and 3.5% high boilers. The mole ratio of the butanal to heptanals is 1.04:1, the mole ratio of the product aldehydes, 2-ethyl-hexenal, 2-undecenals and 2-tetradecenals is 0.84 to 2.51 to 0.65 respectively. The 2-undecenals were separated from the other products by reduced pressure distillation at 40 mm Hg. The 2-undecenals were collected in the temperature range of 119° to 132°. The skeletal structures of the undecenals are given in Table 8.

TABLE 8

| Structure | Compound Name |
|---|---|
| C-C-C-C-C-C-C=C-C-C<br>C* | (E+Z)-2-Ethyl-2-Nonenal |
| C-C-C-C-C-C=C-C-C<br>C C* | (E+Z)-2-Ethyl-7-Methyl-2-Octenal |
| C-C-C-C-C-C=C-C-C<br>C C* | (E+Z)-2-Ethyl-5-Methyl-2-Octenal |
| C<br>C-C-C-C=C-C-C-C-C<br>C* | (E+Z)-2-(1-Methylbutyl)-2-Hexenal |
| C-C-C-C=C-C-C-C-C<br>C* C | (E+Z)-2-(3-Methylbutyl)-2-Hexenal |
| C-C-C-C=C-C-C-C-C-C<br>C* | (E+Z)-2-Pentyl-2-Hexenal |
| C<br>C-C-C-C=C-C-C-C<br>C* C | (E+Z)-2-(1,2-Dimethylpropyl)-2-Hexenal |
| C<br>C-C-C-C-C=C-C-C<br>C C* | (E+Z)-2-Ethyl-4,5-Dimethyl-2-Heptenal |
| C-C-C-C-C-C=C-C-C<br>C C* | (E+Z)-2-Ethyl-4-Methyl-2-Octenal |
| C-C-C-C-C=C-C-C<br>\| C*<br>C-C | (E+Z)-2-Ethyl-4-Ethyl-2-Heptenal |

The 2-undecenals and 2-tetradecenals in the product which are suitable for preparing detergent hydrophobes, can be represented by the following formula:

Structure of $C_8$, $C_{11}$ and $C_{14}$ Enals from Aldol of Butanal and Mixed Heptanals

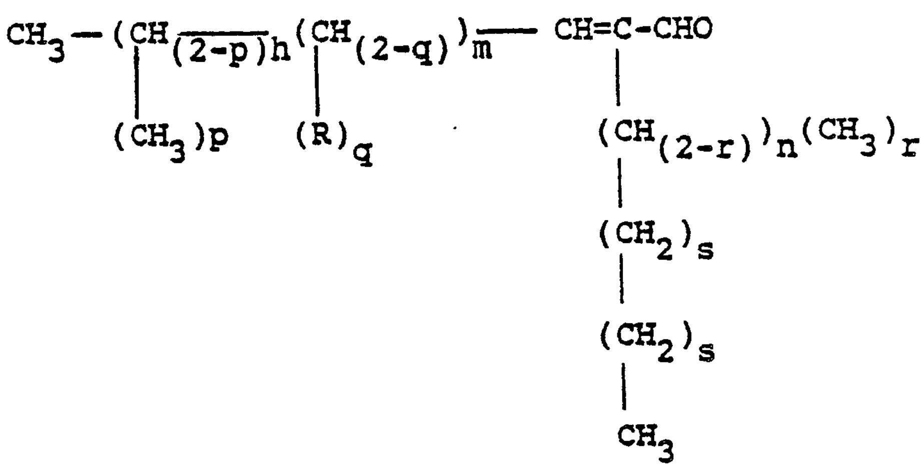

Wherein:

R = methyl or ethyl; h and m = 0, 1, 2, 3, 4 and 5;

n and s = 0, 1, 2; p, q, r and t = 0 or 1; q and p = 0 when m + h = 5 or when m + h = 2 n + s can never be greater than 2 m + h can never be greater than 5;

h = 3 and m = 1 when R = methyl and q = 1 and p = 0;
h = 2 and m = 1 when R = ethyl and q = 1 and p = 0 or when q = 0 and p = 1;
h= 1 and m = 3 when p = 1 and q = 0;
r and t = 0 when n + s = 3 or when n + s = 0;
s = 0 when n, r and t = 1 or when n = 2, r = 0 and t = 1;
s = 1 when n = 1 and t and r = 0 or 1 but only one of t and r can be 1;
s = 2 when t = 1 and n = 0.

When coverted to an alkylated amine, the structure will be as above but with —CHO replaced by $CH_2$—NR'R'' in which R' and R'' are hydrocarbyl or hydroxyhydrocarbyl. Also, hydrogen will have been added to the double bond so that the alkyl group is not unsaturated at the 2-position.

Example 28

This example illustrates the utility of the invention for a single hexene isomer, 4-methylpentene-1. The hydroformylation was performed in a 300 ml autoclave with agitation. The autoclave was charged with 0.29 g carbonyltris(triphenylphosphine)-rhodium hydride, 33.0 g triphenylphosphine, 100.0 g 4-methylpentene-1 and 200 psi (1379 KPa) gauge pressure with 9 parts hydrogen to 1 part CO. The autoclave was heated to 100°C and the pressure brought to 300 psi (2068.4 KPa) gauge using feed gas in 1 to 1 ratio hydrogen to CO. The temperature and pressure were maintained for 2 hours and 45 minutes after which the autoclave and contents were cooled rapidly. The product was removed and analyzed chromatographically. The product consists of 5.65% hexenes (5.57% 4-methylpentene 1), 10.64% 2,4-dimethylpentanal, 68.84% 5-methylhexanal, and approx. 15% triphenylphosphine, by area percent. An aldehyde mixture representative of the oxo reaction of 4-methylpentene-1, as described above was reacted in an aldol condensation. The reaction vessel was a 100 ml 3 neck round bottom flask equipped with pressure equilizing funnel, thermometer, spiral condensor, a mechanical stirrer and a nitrogen blanket, maintained by using a slow bleed of nitrogen and a mineral oil bubbler. The reactor was charged with 12 ml 0.8M NaOH, and 12 ml 2,5-hexanediol, as co-solvent. The system was heated to 80°C and the aldehydes, 12 ml, were added over a six minute period. The system was heated to 89—90°C and held at temperature for an additional 30 minutes.

The system was cooled and the upper and lower phases were separated. The upper phase was analyzed chromatographically and contained 2.0% 2,4-dimethylpentanal, 1.5% 5-methylhexanal, 5% tetradecenal isomers, 85% 7-methyl-2-(3-methylbutyl)-2-octenal, 2.5% 2,5-hexanediol and a balance of high boilers.

A mixture of amylenes and Dimate® hexenes can be subjected to oxo and aldol processes in accord with the present invention. Thus, pentenes containing n-pentene-1, n-pentene-2,3-methylbutene-1, 2-methylbutene-2, and 2-methylbutene-1 in admixture with Dimate® hexenes can be reacted in an oxo reaction with the product treated in an aldol reaction, in accord with procedures in Examples 22 and 23, and the resulting enals (see Table 8) can be represented by the following structure:

Structure of Dodecenals and Tridec-2-enals from the, Oxo-Aldol of mixed pentenes and Dimate Hexenes

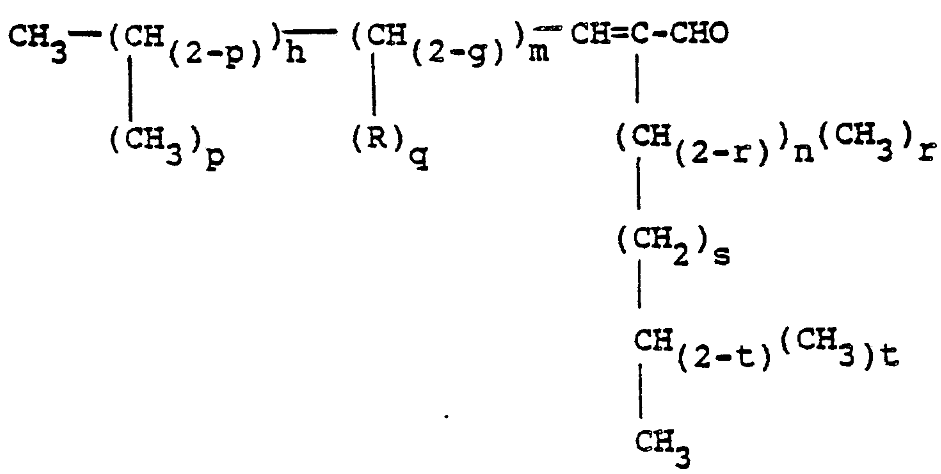

Wherein:
R = methyl or ethyl; h and m = 0, 1, 2, 3, 4 and 5;
n and s = 0, 1, 2; p. q, r and t = 0, 1;
m + h = 5 or 4, when p and q = 0;

m and q = 1 and p = 0 when h = 3, 2 or 1 but R can only be ethyl when h = 1, 2 and R can only be methyl when h = 2, 3;
h + m can never be greater than 5;
n + s can never be greater than 2;
q = 0 and p = 1 and h = 2 when m = 1 or 0;
q = 0 and p = 1 and l = 1 when m = 3 or 2;
r and t = 0 when n + s = 3 or when n = 0 and s = 2;
t = 1 when n = 0 or 1 and s = 1 or 2;
n and t = 1 when s = 0 and r = 1;

There are a number of structural formulae herein illustrating structures of individual aldehydes or mixtures of aldehydes, for example, as immediately hereinabove. All of such aldehydes can be aminated, i.e. can be used to reductively alkylate amines. In the resulting amines, the aldehydes group —CHO (in some cases designated as *C) has been replaced by a $—CH_2—NR'R''$ group, in which R' and R'' are selected from hydrogen, hydrocarbyl, hydroxyhydrocarbyl groups. R' and R'' are generally lower alkyl groups or hydroxyalkyl groups, particularly methyl groups, or one or both can be hydrogen in the event a secondary or primary amine is formed. Also, R' and R'' can be an additional alkyl formed from the aldehyde, as in methyl(ditetradecyl) amine. Also, the double bond at the 2-position of the aldehydes will be saturated in the alkyl groups.

Example 29

This example illustrates conversion of a branched tetradecyl amine to a quaternary ammonium salt. A quantity of N,N-dimethyltetradecylamine from Example 7 (24.1 grams, 0.1 mole) was mixed with benzyl chloride (12.6 grams, 0.1 mole) and charged to a 100 ml reactor at 80°C. No exotherm was observed. After 45 minutes, 1H NMR indicated 20% conversion. The temperature was increased to 90°C and after 2½ hours the reaction mixture was thick and a small second layer formed on top. A mechanical stirrer was substituted for the magnetic stirrer, but the reaction mixture quickly solidified, after a total heating time of three hours. The reaction mixture was permitted to stand an additional hour, cooled and stirred with 200 ml petroleum ether (35°—75° B.P.) for three days and the solids (hydroscopic) were filtered off and dried under vacuum, having a weight of 25.02 grams. An 1H NMR spectrum of the solid dissolved in deuterium oxide ($D_2O$) shows that the $N—CH_3$ and $N—CH_2—$ portions have shifted downfield from 2.1 in the starting amine to 3.2 in the product, characteristic of the formation of quanternary ammonium salt. Resonances at 4.7 and 7.6 represent the benzylic and aromatic protons respectively. Analysis for benzyldimethyltetradecylammonium chloride, calc'd C, 75.06; N 3.80; H, 11.50. Found: C, 74.30; N, 3.66; H, 11.81.

Example 30

A 35 gram amount of dimethyltetradecylamines from Example 7 and 100 ml toluene were sealed in a Parr pressure reactor, which was cooled in a dry ice/acetone bath, and methyl chloride, about 10 ml, was condensed in the reactor. The mixture was heated at 70°C for 15 hours, and then kept at 80°C for the balance of a 64 hour heating period. The reaction mixture was brought to room temperature, the reactor was opened and the mixture was stirred until methyl chloride had boiled off. The toluene was removed by evaporation on a rotary evaporator with water bath heating. The last traces of toluene were removed by vacuum at 1 mm Hg. The crude product was a gum and an NMR in $CdCl_3$ indicated comple conversion to the salt, with no unreacted amine being apparent by NMR. The crude tetradecyltrimethylammonium chloride was dissolved in about 100 ml toluene and extracted into water, with four extractions using a total of about 900 ml water. The water was stripped off, with foaming being encountered, to leave a 43.3 gram residue after overnight vacuum aspiration. An NMR indicated some remaining water of hydration, so the material was heated at 60°C under vacuum (about 1 mm Hg) for several hours, when NMR showed little if any water present. The weight was 38.7 grams for a 90.1% yield of the quaternary salt. The salt was readily soluble in the non-polar solvent, such as petroleum ether (30 to 75°C B.P.) and also toluene. This contrasts with reported low solubility in polar solvents at room temperature of dodecyltrimethylammonium, chloride and octadecylmethylammonium chloride in n-hexane, cyclohexane, benzene and ethyl acetate; see R. A. Reck et al., Solubilities of Dodecyl- and Octadecyl-trimethylammonium Chlorides in Organic Solvents; Journal of Organic Chemistry, Vol. 12, page 517 (1947).

Example 31

A mixture of dimethyltetradecylamines from Example 7, 1.2072 grams, was mixed with 1.71 grams of 30% aqueous hydrogen peroxide and 15 ml methanol, and the mixture was stirred at room temperature for 64 hours. Platinum black (several milligrams) was added to decompose excess hydrogen peroxide. The solution was filtered through filter aid (Celite) in a sintered glass funnel and was concentrated by heating on a rotary evaporator and then under high vacuum. An NMR analysis indicated the reaction had almost reached completion, with only about 5% of the amine unreacted. The amine oxide product was readily soluble in petroleum ether. The product was extracted from the petroleum ether into water, and was then isolated by stripping off the water on a rotary evaporator with water bath heating, and under vacuum (about 1 mm Hg) with further water bath heating for about 1 hour. An NMR analysis in $CdCl_3$ and in

dimethysulfoxide was found that the unreacted amine had been eliminated and that the dimethyltetradecylammonium oxide had formed the dihydrate. The product was obtained in acceptable quantity by the preparation and isolation procedure. The identity of the product is based upon the 'H NMR chemical shifts. The reaction was slow, possibly due to dilute solution employed, but may not have required the 64-hour period which gave a near quantitative yield.

The

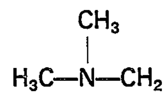

hydrogens were observed to be shifting from δ 2.13 to δ 3.17 in $CdCl_3$ when the amine oxide was formed. The product was found to have high solubility in both polar and non-polar solvents. For example, it can be dissolved readily in water and in petroleum ether (30—75°C b.p.),

Tetradec-2-enals prepared in accord with the invention were converted to corresponding amines as described in Examples 32 and 33 below.

Example 32

This example illustrates the reductive alkylation of amino ethanol with tetradec-2-enals. The tetradec-2-enals, 105.2 grams, along with 3.0 grams of 5% Pd/C catalyst and 13.4 grams of amino ethanol were added to a 300 ml H.P. Magnedrive autoclave under a nitrogen blanket. The autoclave was purged and pressured to 500 psig (3447 KPa gauge) of hydrogen gas. The reaction was started by starting the agitation and setting agitator speed at 1500 rpm. The temperature was brought to 90°C and held until the reaction was completed after 1 hour and 10 minutes. The product was filtered and distilled under vacuum to yield 64.8 grams of pure N,N-ditetradecylamino ethanol. In this procedure and in Example 33 below there was considerable loss of material by absorption in the powdered catalyst used. This could be avoided by use of pelleted catalyst or more careful filtration.

Example 33

This example illustrates the reductive alkylation of N,N-diethanol amine with tetradec-2-enals. The tetradec-2-enals, 92.8 grams, along with 3.0 grams H.P. Magnedrive autoclave under a nitrogen blanket. The autoclave was purged and pressured to 500 psig (3447 KPa gauge) of hydrogen gas. The reaction was started by starting the agitation and setting agitator speed at 1500 rpm. The reaction held at 90°C and was completed after 24 hours and 15 minutes. The product was filtered and distilled under vacuum to yield 101.4 grams of pure N,N-diethanol-tetradecyl amines which came over at 183°C to 188°C and 1.2 mm Hg.

Example 34

This example illustrates the oxidation of N,N-diethanol-tetradecyl amines to the amine oxides. A mixture of the N,N-diethanol-tetradecyl amines from Example 33, 15.0 grams, was mixed with 50 milliliters of isopropanol, 10 milliliters of water and 0.015 grams ethylene diamine tetraacetic acid, disodium salt. Aqueous hydrogen peroxide, 6.20 grams of 30% solution, was added under a nitrogen purge while maintaining the temperature at 60°C. The reaction was held at temperature and agitated for a total of 42 hours and 47 minutes. Platinum black (several milligrams) was added to decompose excess hydrogen peroxide. The platinum was filtered off and the solution had 50 milliliters of water added and pH adjusted to 10.25 ± 2.5 and the solvent removed using a rotary evaporator. Yield was ~100% of the N,N-diethanol-tetradecyl amine oxide by NMR analysis.

Example 35

An aldehyde mixture representative of that from the oxo reaction of Dimate hexenes, as described in Example 1, was reacted in an aldol condensation. A stirred 300 ml autoclave was charged with 50 ml of 5.0M NaOH, 50 ml of methanol and 20 psig nitrogen. The autoclave was purged of air using nitrogen. After heating to 110°C the autoclave had 50 ml, 40.52 grams, of aldehyde and 4.76 grams of decane used as internal standard for analysis pressured into the autoclave with nitrogen. The reaction was run for forty minutes and the system was then rapidly cooled. The product was removed and the upper and lower phases were separated. Analysis of the upper product using an internal standard GC method, quantitative, indicates 90% of all aldehydes were converted to tetradec-2-enals with greater than 99% selectivity.

Certain of the products of the present invention were evaluated to determine detersive efficiency in comparison with reference compounds which were amine oxides with substantially linear long chain alkyl substituents, produced by processes other than those of the present invention. The tests employed include recognized tests, including a test in which a fabric soiled with synthetic sebum/airborne particulate is washed and the results measured by ΔRd, change in reflectance by Gardner XL—23 Color difference

meter; and a related test in which the fabric has been soiled with clay. Tests were conducted with a polyester-cotton blend fabric containing 65% polyester, and with a cotton broadcloth fabric. Tests included tests with a builder system including sodium tripolyphosphate (STP), which generally is used in 18 parts to 9.6 parts of R.U. silicate and 0.75 parts carboxymethylcellulose, and along with 15 parts $Na_2SO_4$. When the STP is omitted, it is replaced by additional $Na_2SO_4$. The detergent was used in concentration of 0.15% so that 10% of the active surfactant, for example, provides 150 ppm of the active surfactant. Results are reported in Table 9.

TABLE 9

| | | | Soiled With Synthetic Sebum | | | |
|---|---|---|---|---|---|---|
| Fabric-65-35 Polyester/Cotton Surfactant | Builder | Water Hardness | 10% | Rd at 15% | 20% | Temp. °F |
| LAS(A—225) | STP | 50 | 14.4 | | 20.3 | 72 |
| SCHERCAMOX | STP | 50 | 22.8 | | 23.5 | 72 |
| Dimethyl-n-tetradecylamine oxide | STP | 50 | 16.9 | | 19.6 | 72 |
| Dimethyl-tetradecylamine oxides | STP | 50 | 18.2 | | 21.9 | 72 |
| Dimethyl-dodecylamine oxides | STP | 50 | 12.1 | | 14.6 | 72 |
| Diethanol-tetradecylamine oxides | STP | 50 | 21.5 | | 21.8 | 72 |
| Diethyl-tetradecylamine oxides | STP | 50 | 16.4 | | 17.6 | 72 |
| LAS(A—225) | STP | 50 | 19.1 | | 20.2 | 105 |
| SCHERCAMOX | STP | 50 | 23.3 | | 24.6 | 105 |
| Dimethyl-n-tetradecylamine oxide | STP | 50 | 16.7 | | 19.3 | 105 |
| Dimethyl-tetradecylamine oxides | STP | 50 | 20.6 | | 22.7 | 105 |
| Diethyl-tetradecylamine oxides | STP | 50 | 17.0 | | 19.7 | 105 |
| LAS | | 150 | 9.1 | | 15.2 | 72 |
| SCHERCAMOX | | 150 | 12.1 | | 23.4 | 72 |
| Dimethyl-n-tetradecylamine oxide | | 150 | 14.8 | | 19.2 | 72 |
| Dimethyl-tetradecylamine oxides | | 150 | 1.3 | | 2.6 | 72 |
| Dimethyl-dodecylamine oxides | | 150 | 1.3 | | 2.2 | 72 |
| Diethanol-tetradecylamine oxides | | 150 | 2.0 | | 4.7 | 72 |
| Diethyl-tetradecylamine oxides | | 150 | 1.6 | | 5.1 | 72 |
| SCHERCAMOX | STP | 150 | | 16.0 | | 72 |
| SCHERCAMOX | STP | 100 | | 17.0 | | 72 |
| SCHERCAMOX | STP | 50 | | 19.9 | | 72 |
| SCHERCAMOX | | 150 | | 13.3 | | 72 |
| SCHERCAMOX | | 100 | | 15.7 | | 72 |
| SCHERCAMOX | | 50 | | 14.3 | | 72 |

TABLE 9 continued

| Fabric-65-35 Polyester/Cotton Surfactant | Builder | Water Hardness | Soiled With Synthetic Sebum 10% | Rd at 15% | 20% | Temp. °F |
|---|---|---|---|---|---|---|
| Dimethyl-tetradecylamine oxides | STP | 150 | | 16.5 | | 72 |
| Fabric Cotton | | | Soiled With Clay | | | |
| Dimethyl-tetradecylamine oxides | STP | 100 | | 17.0 | | 72 |
| Dimethyl-tetradecylamine oxides | STP | 50 | | 19.9 | | 72 |
| Dimethyl-tetradecylamine oxides | | 150 | | 14.1 | | 72 |
| Dimethyl-tetradecylamine oxides | | 100 | | 14.8 | | 72 |
| Dimethyl-tetradecylamine oxides | | 50 | | 16.3 | | 72 |

Rd is delta reflectance, % is percentage active surfactants in mix tested. Water hardness is in ppm and is 100% $Ca^{++}$ based as $CaCO_3$. LAS(A—225) is a Linear Alkybenzene Sulfonate as a sodium salt a commercially used surfactant.

SCHERCAMOX is a linear dimethyl-dodecyl oxide sold commercially as a surfactant. Dimethyl-n-tetradecyl amine oxide is a linear tetradecylamine oxide. All other amine oxides are mixed isomers of the long alkyl chain formed as described earlier through hydroformylation followed by aldol condensation.

It can be seen that the dodecylamine and tetradecyl amine oxides of the present invention give good results in the test with synthetic sebum soil, at low water hardness, even at moderate temperature, but poor results at high water hardness, while results are good at both high and low hardness in the test with clay soil. It has not been determined whether the poor results at high hardness in the synthetic sebum test are due to some impurity or involve a fundamental property. However, some foam screening tests indicate the compounds have sudsing and foam stability properties in the presence of fatty soil which generally correlate with good detersive properties, particularly suggesting application in light duty liquid detergents, such as detergents for washing dishes.

Dimethyldodecylamine oxides having properties as reported in Table 9 is obtainable from aldol reactions of pentanals and heptanals as reported in Example 23, followed by use of the appropriate cut in amination and oxidation reactions as taught herein. Similar results are obtainable from aldol reactions of hexanals, followed by conversion to amine oxides. The tetradecylamine oxides tested were obtained by aldol of heptanals from hexenes, and subsequent conversion to amine oxides, as described and illustrated herein.

The various amines disclosed herein can readily be converted to corresponding amine oxides and quaternary ammonium salts, in accord with procedures taught herein. Such amine oxide and salts will find use in surfactant, detergent and bacteriostat applications in which amine oxide and quaternary ammonium salt compounds are presently used or known to be useful, and may find expanded or additional such uses in view of the efficient synthesis routes for their preparation which are illustrated herein.

Example 36

In this example, the procedures used to obtain the data in Table 10 are described. In all runs 1 gram of catalyst, 115 grams of 2-pentylnon-2-enal prepared, as described in Example 14 above, and 30 grams of anhydrous dimethylamine were used. The above reactants were placed into a 300 ml stirred autoclave and the autoclave was sealed. The system was pressured to 500 psig (18447 KPa gauge) with nitrogen and the nitrogen vented off to atmospheric pressure. The system was then re-pressurized to 500 psig (63447 KPa gauge) with the hydrogen vented again. The system was then pressurized to reaction pressure, see Table 10, and heated to the temperature indicated under column Temp. A sample was taken at the end of the run. All products were first reacted with hydroxylamine hydrochloride to form the oxime derivatives of the amine products and these were then reacted with trimethylchlorosilane to form the trimethylsilyl derivatives which were analyzed in a gas chromatograph on an OV—1 glass capillary column. The analytical procedure used is published in the Journal of Chromatographic Science, Vol. 21, 132—138, (March, 1983).

TABLE 10

Reductive Amination Catalysts Results

| Catalyst | Temp C | Pressure gauge kPa | Area Percents en-amine | an-amine | enol | anol | enal | anal | others |
|---|---|---|---|---|---|---|---|---|---|
| Calsicat e-106tr Cu—Cr powder | 150 | 6895 | 44.0 | 13.7 | 34.3 | 8.0 | tr | tr | — |
| Harshaw Ni-3210 powder | 150 | 6895 | 5.8 | 48.4 | 0.9 | 7.8 | 2.1 | 34.6 | — |
| UCI G—62 Co powder | 150 | 6895 | 11.0 | 23.7 | 17.0 | 48.3 | tr | tr | tr |
| Harshaw Co—016h ⅛" tab | 150 | 3447 | 2.2 | — | 2.0 | 1.7 | 77.6 | 14.3 | 2.1 |
| Harshaw Ni—3210 ⅛" tab | 120 | 3447 | 1.7 | 20.4 | 0.6 | 5.3 | 36.6 | 35.0 | 0.8 |

In Table 10, the Area Percents are the relative amounts of the designated types of compounds present in the sample at the end of the run, with en-amine and an-amine being the desired dimethyl(2-pentylnon-2-enyl) amine and dimethyl(2-pentylnonyl)amine, enol and anol being 2-pentylnon-2-enol and 2-pentylnonenol, and enal being the starting 2-pentylnon-2-enal, and anal the starting compound with the olefin group reduced, 2-pentylnonanal.

**Claims**

1. A composition comprising an amine having a higher branched-chain alkyl group attached to the amino nitrogen, said branched alkyl group having 10 to 18 carbon atoms and an alkyl branch containing 3 to 7 carbon atoms at the 2-position, characterised in that (a) the composition comprises a mixture of such amines, (b) most of the amines have additional branching in said higher alkyl group and (c) most of the additional branches are methyl or ethyl groups.

2. A composition according to Claim 1, in which most of the additional branches are methyl groups.

3. A composition according to Claim 1 or Claim 2, in which the amines are tertiary amines.

4. A composition according to any of the preceding claims, in which the branched-chain alkyl group has more than two branches in addition to that at the 2-position.

5. A composition according to any of the preceding claims, in which higher branched-chain alkyl groups of different carbon numbers are present.

6. A composition according to any of the preceding claims, comprising amines having two of the higher branched chain alkyl groups.

7. A composition according to Claim 1, comprising a mixture of isomeric amines having a structure:

$$R—CHCH_2NR''R'''$$
$$|$$
$$R^1$$

in which R is an alkyl group of 7 carbon atoms, R' is an alkyl group of 5 carbon atoms, with most of the

isomers having additional methyl branches, and wherein R'' and R''' are hydrogen, hydrocarbyl or hydroxyhydrocarbyl groups.

8. A composition according to Claim 7, in which in at least about 80% of the amines, R' is selected from n-pentyl, 3-methylbutyl and 1-methylbutyl, and R is selected from n-heptyl, 3-methylhexyl, 5-methylhexyl, 2-methylhexyl and 2-ethylpentyl groups; and R'' and R''' are alkyl or hydroxyalkyl groups.

9. A composition according to Claim 7, in which 40 to 60% by weight of the amines have an alkyl substituent selected from 2-(3-methylbutyl)-5-methyloctyl, 2-(1-methylbutyl)-5-methyloctyl and 2-pentyl-5-methyloctyl.

10. A composition according to Claim 7, in which 65% to 80% by weight of the amines are comprised of the amines in Claim 7, along with amines having 2-(3-methylbutyl)-7-methyloctyl, 2-pentylnonyl, 2-pentyl-7-methyloctyl and 2-(3-methylbutyl)-nonyl substituents.

11. A composition according to either Claim 1 or Claim 3, in which most of the amines have an alkyl substituent represented by the structure:

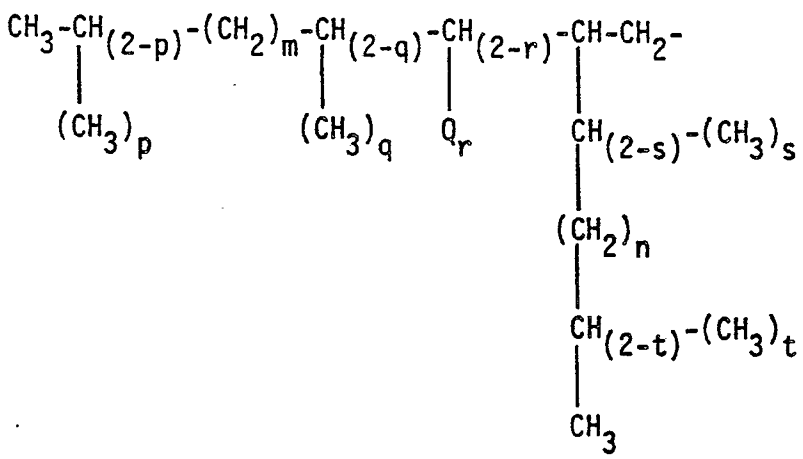

wherein:

Q = methyl or ethyl;
p, q and r = 0 or 1, but only one of p, q and r can be 1;
m = 3, when p, q and r = 0;
m = 2, when r = 1 and Q = methyl;
m = 1, when r = 1 and Q = ethyl;
n = 2, when s and t = 0
n = 1, when s or t = 1;
s and t = 0 or 1, but only one of s and t can be 1.

12. A mixture of isomeric amines according to either Claim 1 or Claim 3, and further characterised in that most of the amines in the mixture have an alkyl group represented by the structure:

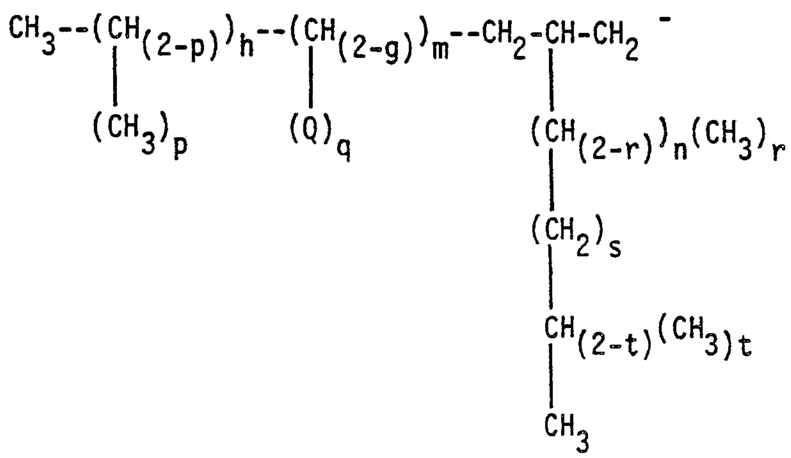

Wherein:

Q = methyl or ethyl; h and m = 0, 1, 2, 3, 4 and 5
n and s = 0, 1, 2; p, q, r and t = 0 or 1;
m + h can never = more than 5

q and p = 0 when m + h = 5 or when m + h = 3
h = 3 and m = 1 when Q = methyl and q = 1 and p = 0
q = 0 and p = 1 when h = 2 and m = 2 or when h = 1 and m = 3;
n and s can never = more than 3
r and t = 0 when n + s = 3 or when n + s = 1;
s = 0 when n, r and t = 1 or when n = 2 and r = 0 and t = 1
s = 1 when n = 1 and t and r = 0 or 1 but only one of t and r can be 1;
s = 2 when t = 1 and n = 0
h = 2 and m = 1 when Q = ethyl and q = 1 and p = 0.

13. A composition comprising amine oxide or quaternary ammonium salt derivatives of a mixture of amines as defined in any of the preceding claims.

14. A process for producing a mixture of amines having an N-alkyl substituent with 10 to 18 carbon atoms from olefin feedstock, which comprises reacting an olefin of 4 to 8 carbon atoms with carbon monoxide and hydrogen to obtain aldehydes having 5 to 9 carbon atoms, subjecting such aldehydes to a base-catalyzed aldol reaction to produce aldol product aldehydes of 10 to 18 carbon atoms, and reacting such aldol product aldehydes with an amine or ammonia under reductive alkylation conditions to produce amines with an N-alkyl substituent of 10 to 18 carbon atoms.

15. A process of Claim 14, in which the aldol product aldehydes are reacted with an amine to produce tertiary amines with alkyl substituent of 10 to 18 carbon atoms.

16. A process of either Claim 14 or Claim 15, in which the amine forming reaction is effected under hydrogen pressure in contact with a catalyst for reduction.

17. A process of Claim 16, in which the catalyst is a metal or metal oxide catalyst.

18. A process of Claim 16, in which the catalyst is a noble metal.

19. A process of any of Claims 14 to 18, in which the aldol product is reacted with a dialkylamine in which the alkyl groups each have 1—4 carbon atoms.

20. A process of any of Claims 14 to 19, in which the $C_{5-9}$ aldehydes are comprised of at least 50% alkanals with no branching at the 2-position and the aldol product contains no more than 20% of that produced from cross-aldol of 2-substituted aldehydes.

21. A process of any of Claims 14 to 29, in which the aldol product is reacted in enal form.

22. A process of any of Claims 14 to 21, in which the aldol reaction is conducted in aqueous alkali and there is present a cosolvent that aids in improving contact of the aldehydes with the aqueouus alkali.

23. A process of Claim 22, in which a hydroxyl compound is employed as cosolvent.

24. A process of any of Claims 14 to 23, in which the aldol product is hydrogenated to saturated aldehydes and then reacted to produce amines.

25. A process of any of Claims 14 to 24, in which the oxo reaction is conducted with cobalt catalyst at a temperature in the range of 80 to 150°C and a pressure sufficient to maintain catalyst stability but not over 5000 psi (34,475 kPa), and the aldol reaction is conducted in aqueous alkaline medium at a temperature of 90° to 130°C.

26. A process of Claim 19, in which aldehydes for the aldol reaction have a content of alkanals with no 2-branching of 60 to 80%, and the conversion of these aldehydes to aldol product is in the range of 75% to 90%.

27. A process of Claim 14 for preparing amines with detergent range hydrophobe groups which comprises conducting an oxo reaction with hydrogen, carbon monoxide and olefins selected from those having 5 to 7 carbon atoms to obtain aldehydes having from 6 to 8 carbon atoms and comprised of at least 50% alkanals with no branching at the 2-position and subjecting the aldehydes to an aldol reaction to cause good conversion to aldol product but with that produced from cross-aldol of 2-substituted aldehydes consisting of no more than 20% of the product and reactive aldol product aldehydes with an amine of $NH_3$ under reductive conditions to produce amines with an N-alkyl substituent corresponding to the aldol product aldehyde.

28. A process of Claim 27 in which the aldol reaction is conducted in aqueous alkali and there is present a cosolvent that aids in improving contact of the aldehydes with the aqueous alkali.

29. A process of Claim 14 for preparing amines with a high content of N-alkyl substituents in the $C_{11}$ to $C_{14}$ range, which comprises conducting an aldol reaction of butanal with heptanals obtained from oxo reaction of branched hexenes from propylene dimerization, and obtaining an aldol product with a high content of 2-undecenals and subjecting aldol product aldehyde to reaction with an amine under reductive alkylation conditions.

30. A process according to Claim 29 of preparing amines with $C_{14}$ alkyl substituents which comprises conducting an oxo reaction with hydrogen carbon monoxide and a hexene mixture comprised mainly of methylpentenes with internal unsaturation with no more than 30% linear hexenes so as to obtain $C_7$ aldehydes with less than 30% 2-substituted aldehydes and subjecting the aldehydes to aldol reaction to obtain aldol product aldehydes with conversion of at least 70% and reacting aldol product aldehyde with a dialkyl amine under reductive conditions to produce a $C_{13}$-alkyl-substituted amine.

31. A process of any of Claims 14 to 30, in which the reaction with an amine is at a temperature no greater than 50°C.

32. A process of Claim 31, in which the reaction with amine utilizes a palladium catalyst under hydrogen pressure.

33. A process of Claim 27 in which the reaction with an amine is at a temperature in the range of 35° to 80°C.

34. A process of Claim 30, in which the hexene mixture comprises substantially the hexenes mixture from a dimerization of propylene over a nickel and aluminium alkyl catalyst.

**Patentansprüche**

1. Zusammensetzung umfassend ein Amin mit einer am Aminostickstoff hängenden höheren verzweigtkettigen Alkylgruppe, welche verzweigte Alkylgruppe 10 bis 18 Kohlenstoffatome und eine Alkylabzweigung mit 3 bis 7 Kohlenstoffatomen in Stellung 2 aufweist, dadurch gekennzeichnet, daß (a) die Zusammensetzung eine Mischung derartiger Amine umfaßt, (b) die meisten der Amine eine zusätzliche Abzweigung in der genannten höheren Alkylgruppe aufweisen und (c) die meisten der zusätzlichen Abzweigungen Methyl- oder Äthylgruppen sind.

2. Zusammensetzung nach Anspruch 1, in der die meisten der zusätzlichen Abzweigungen Methylgruppen sind.

3. Zusammensetzung nach Anspruch 1 oder 2, in der die Amine tertiäre Amine sind.

4. Zusammensetzung nach einem der vorhergehenden Anspruche, in der die verzweigkettige Alkylgruppe mehr als zwei Abzweigungen zusätzlich zu jener in Stellung 2 aufweist.

5. Zusammensetzung nach einem der vorhergehenden Anspruche, in der höhere verzweigtkettige Alkylgruppen mit verschiedenen Kohlenstoffatomzahlen vorhanden sind.

6. Zusammensetzung nach einem der vorhergehenden Anspruche umfassend Amine mit zwei der höheren verzweigtkettigen Alkylgruppen.

7. Zusammensetzung nach Anspruch 1 umfassend eine Mischung von isomeren Aminen der Formel

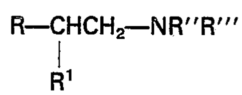

worin R eine Alkylgruppe mit 7 Kohlenstoffatomen bedeutet und R′ eine Alkylgruppe mit 5 Kohlenstoffatomen darstellt, wobei die meisten der Isomere zusätzliche Methylabzweigungen aufweisen, und worin R″ und R‴ Wasserstoff, Hydrocarbyl- oder Hydroxyhydrocarbylgruppen sind.

8. Zusammensetzung nach Anspruch 7, in der in mindestens etwa 80% der Amine R′ ausgewählt ist aus n-Pentyl, 3-Methylbutyl und 1-Methylbutyl und R ausgewählt ist aus n-Heptyl-, 3-Methylhexyl-, 5-Methylhexyl-, 2-Methylhexyl- und 2-Äthylpentylgruppen und R″ und R‴ Alkyl- oder Hydroxyalkylgruppen sind.

9. Zusammensetzung nach Anspruch 7, in der 40 bis 60 Gew.% der Amine einen Alkylsubstituenten ausgewählt aus 2-(3-Methylbutyl)-5-methyloctyl, 2-(1-Methylbutyl)-5-methyloctyl und 2-Pentyl-5-methyloctyl aufweisen.

10. Zusammensetzung nach Anspruch 7, in der 65 bis 80 Gew.% der Amine aus den Aminen in Anspruch 7 zusammen mit Aminen mit 2-(3-Methylbutyl)-7-methyloctyl-, 2-Pentylnonyl-, 2-Pentyl-7-methyloctyl- und 2-(3-Methylbutyl)-nonyl-Substituenten bestehen.

11. Zusammensetzung nach Anspruch 1 oder 3, in der die meisten der Amine einen Alkylsubstituenten dargestellt durch die Struktur

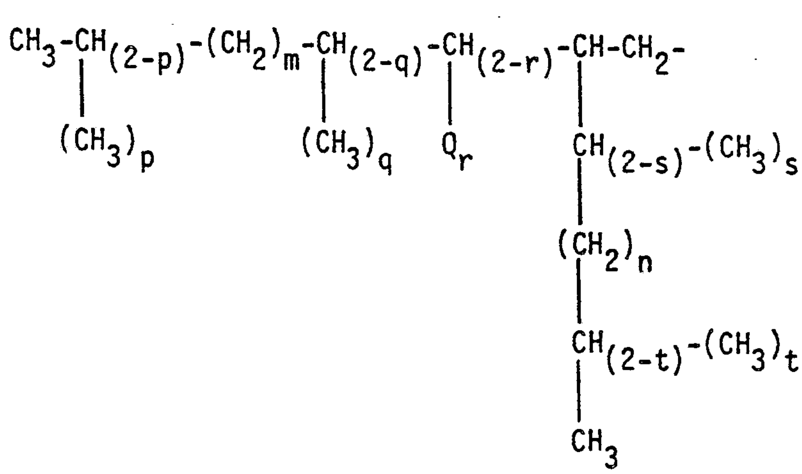

aufweisen, worin:

Q = Methyl oder Äthyl,

p, q und r = Null oder 1, aber nur eines von p, q und r 1 sein kann,
m = 3, wenn p, q und r = Null,
m = 2, wenn r = 1 und Q = Methyl,
m = 1, wenn r = 1 und Q = Äthyl,
n = 2, wenn s und t = Null,
N = 1, wenn s oder t = 1,
s und t = Null oder 1, aber nur eines von s und t 1 sein kann.

12. Mischung von isomeren Aminen nach Anspruch 1 oder 3, weiters dadurch gekennzeichnet, daß die meisten der Amine in der Mischung eine Alkylgruppe dargestellt durch die Struktur

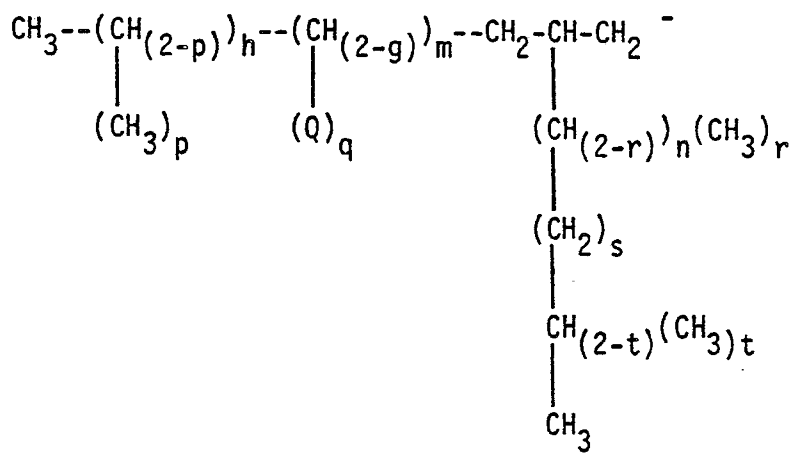

aufweisen, worin:
Q = Methyl oder Äthyl; h und m = Null, 1, 2, 3, 4 oder 5;
n und s = Null, 1 oder 2; p, q, r und t = Null oder 1;
m + h niemals mehr als 5 sein können;
q und p = Null, wenn m + h = 5 oder wenn m + b = 3;
h = 3 und m = 1, wenn Q = Methyl und q = 1 und p = Null;
q = Null und p = 1, wenn h = 2 und m = 2 oder wenn h = 1 und m = 3;
n und s niemals mehr als 3 sein können;
r und t = Null, wenn n + s = 3 oder wenn n + s = 1;
s = Null, wenn n, r und t = 1 oder wenn n = 2 und r = Null und t = 1;
s = 1, wenn n = 1 und t und r = Null oder 1, aber nur eines von t und r 1 sein kann;
s = 2, wenn t = 1 und n = Null;
h = 2 und m = 1, wenn Q = Äthyl und q = 1 und p = Null.

13. Zusammensetzung umfassend Aminoxid- oder quaternäre Ammoniumsalzderivate einer Mischung von Aminen, wie in einem der vorhergehenden Ansprüche definiert.

14. Verfahren zur Herstellen einer Mischung von Aminen mit einem N-Alkylsubstituenten mit 10 bis 18 Kohlenstoffatomen aus einem Olefin-Ausgangsmaterial, welches umfaßt, daß ein Olefin mit 4 bis 8 Kohlenstoffatomen mit Kohlenmonoxid und Wasserstoff zum Erzielen von Aldehyden mit 5 bis 9 Kohlenstoffatomen umgesetzt wird, derartige Aldehyde einer basenkatalysierten Aldol-Reaktion zur Bildung von Aldolproduktaldehyden mit 10 bis 18 Kohelnstoffatomen unterworfen werden und derartige Aldolproduktaldehyde mit einem Amin oder Ammoniak unter reduktiven Alkylierungsbedingungen zum Bilden von Aminen mit einem N-Alkylsubstituenten mit 10 bis 18 Kohlenstoffatomen umgesetzt werden.

15. Verfahren nach Anspruch 14, in dem die Aldolproduktaldehyde mit einem Amin zur Bildung von tertiären Aminen mit einem Alkylsubstituenten mit 10 bis 18 Kohlenstoffatomen umgesetzt werden.

16. Verfahren nach Anspruch 14 oder 15, in dem die Aminbildungsreaktion unter Wasserstoffdruck in Berührung mit einem Katalysator für die Reduktion bewirkt wird.

17. Verfahren nach Anspruch 16, in dem der Katalysator ein Metall- oder Metalloxidkatalysator ist.

18. Verfahren nach Anspruch 16, in dem der Katalysator ein Edelmetall ist.

19. Verfahren nach einem der Ansprüche 14 bis 18, in dem das Aldolprodukt mit einem Dialkylamin umgesetzt wird, in dem die Alkylgruppen jeweils 1 bis 4 Kohlenstoffatome aufweisen.

20. Verfahren nach einem der Ansprüche 14 bis 19, in dem die $C_{5-9}$-Aldehyde aus mindestens 50% Alkanalen ohne Verzweigung in Stellung 2 bestehen und das Aldolprodukt nicht mehr als 20% des von der gegenseitigen Aldol-Reaktion von 2-substituierten Aldehyden gebildeten Produktes enthält.

21. Verfahren nach einem der Ansprüche 14 bis 29, in dem das Aldolprodukt in Enalform umgesetzt wird.

22. Verfahren nach einem der Ansprüche 14 bis 21, in dem die Aldol-Reaktion in wässerigem Alkali

durchgeführt wird und ein Colösungsmittel vorhanden ist, das zum Verbessern des Kontaktes der Aldehyde mit dem wässerigen Alkali beiträgt.

23. Verfahren nach Anspruch 22, in dem eine Hydroxylverbindung als Colösungsmittel verwendet wird.

24. Verfahren nach einem der Ansprüche 14 bis 23, in dem das Aldolprodukt zu gesättigten Aldehyden hydriert und dann zur Bildung von Aminen umgesetzt wird.

25. Verfahren nach einem der Ansprüche 14 bis 24, in dem die Oxo-Reaktion mit einem Kobaltkatalysator bei einer Temperatur im Bereich von 80 bis 150°C und einem Druck, der ausreichend ist, die Katalysatorstabilität aufrechtzuerhalten, aber nicht uber 5000 psi (34 475 kPa) durchgeführt wird und die Aldol-Reaktion in wässerigem alkalischen Medium bei einer Temperatur von 90 bis 130°C durchgeführt wird.

26. Verfahren nach Anspruch 19, in dem Aldehyde für die Aldol-Reaktion einen Gehalt an Alkanalen ohne 2-Verzweigung von 60 bis 80% aufweisen und die Überführung dieser Aldehyde in das Aldolprodukt im Bereich von 75 bis 90% liegt.

27. Verfahren nach Anspruch 14 zur Herstellung von Aminen mit hydrophoben gruppen im Detergensbereich, welches umfaßt, daß eine Oxo-Reaktion mit Wasserstoff, Kohelnmonoxid und Olefinen ausgewählt aus jenen mit 5 bis 7 Kohlenstoffatomen durchgeführt wird, um Aldehyde mit 6 bis 8 Kohlenstoffatomen bestehend aus mindestens 50% Alkanalen ohne Verzweigung in Stellung 2 zu erhalten, und die Aldehyde einer Aldol-Reaktion unterworfen werden, um eine gute Überführung in das Aldolprodukt zu bewirken, wobei aber das durch die gegenseitige Aldol-Reaktion von 2-substituierten Aldehyden gebildete nicht mehr als 20% des Produktes enthält, und Aldolproduktaldehyde mit einem Amin oder $NH_3$ unter reduktiven Bedingungen umgesetzt werden, um Amine mit einem N-Alkylsubstituenten entsprechend dem Aldolproduktaldehyd zu bilden.

28. Verfahren nach Anspruch 27, in dem die Aldol-Reaktion in wässerigem Alkali durchgeführt wird und ein Colösungsmittel vorhanden ist, das zum Verbessern des Kontaktes der Aldehyde mit dem wässerigen Alkali beiträgt.

29. Verfahren nach Anspruch 14 zum Herstellen von Aminen mit einem hohen Gehalt an N-Alkylsubstituenten im $C_{11}$—$C_{14}$-Bereich, welches umfaßt, daß eine Aldol-Reaktion von Butanal mit Heptanalen erhalten aus der Oxo-Reaktion von verzweigten Hexenen von der Propylendimerisierung durchgeführt und ein Aldolprodukt mit einem hohen Gehalt an 2-Undecenalen erhalten wird und der Aldolproduktaldehyd mit einem Amin unter reduktiven Alkylierungsbedingungen umgesetzt wird.

30. Verfahren nach Anspruch 29 zum Herstellen von Aminen mit $C_{14}$-Alkylsubstituenten, welches umfaßt, daß eine Oxo-Reaktion mit Wasserstoff und Kohlenmonoxid und einer Hexenmischung, die hauptsächlich aus Methylpentenen mit innerer Ungesättigtheit mit nicht mehr als 30% linearen Hexenen besteht, durchgeführt wird, um $C_7$-Aldehyde mit weniger als 30% 2-substituierten Aldehyden zu erhalten, und die Aldehyde der Aldol-Reaktion unterworfen werden, um Aldolproduktaldehyde mit einer Überführung von mindestens 70% zu erhalten, und der Aldolproduktaldehyd mit einem Dialkylamin unter reduktiven Bedingungen umgesetzt wird, um ein $C_{13}$-alkylsubstituiertes Amin zu erhalten.

31. Verfahren nach einem der Ansprüche 14 bis 30, in dem die Reaktion mit einem amin bei einer Temperatur von nicht höher als 50°C durchgeführt wird.

32. Verfahren nach Anspruch 31, in dem die Reaktion mit Amin einen Palladiumkatalysator unter Wasserstoffdruck verwendet.

33. Verfahren nach Anspruch 27, in dem die Reaktion mit einem Amin bei einer temperatur im Bereich von 35 bis 80°C durchgeführt wird.

34. Verfahren nach Anspruch 30, in dem die Hexenmischung im wesentlichen die Hexenmischung von einer Dimerisierung von Propylen über einem Nickel- und Aluminiumalkylkatalysator umfaßt.

**Revendications**

1. Composition comprenant une amine ayant un groupe alkyle à chaîne ramifiée supérieure fixée à l'atome d'azote, ce groupe alkyle ramifié ayant 10 à 18 atomes de carbone et une ramification alkyle contenant 3 à 7 atomes de carbone à la position 2, caractérisée en ce que (a) la composition comprend un mélange de telles amines, (b) la plupart des amines ont une ramification supplémentaire dans le groupe alkyle supérieur et (c) la plupart des ramifications supplémentaires sont des groupes méthyle ou éthyle.

2. Composition selon la revendication 1, dans laquelle la plupart des ramifications supplémentaires sont des groupes méthyle.

3. Composition selon la revendication 1 ou la revendication 2, dans laquelle les amines sont des amines tertiaires.

4. Composition selon l'une quelconque des revendications précédentes, dans laquelle le groupe alkyle à chaîne ramifiée comporte plus de deux ramifications en plus de celle à la position 2.

5. Composition selon l'une quelconque des revendications précédentes, dans laquelle des groupes alkyle à chaîne ramifiée supérieure de nombres de carbone différents sont présents.

6. Composition selon l'une quelconque des revendications précédentes, comprenant des amines ayant deux des groupes alkyle à chaîne ramifiée supérieure.

7. Composition selon la revendication 1, comprenant un mélange d'amines isomériques ayant une structure:

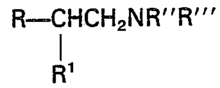

dans laquelle R est un groupe alkyle en $C_7$, R' est un groupe alkyle en $C_5$, avec la plupart des isomères ayant des ramifications méthyle supplémentaires, et dans laquelle R'' et R''' sont l'hydrogène, des groupes hydrocarbyle ou hydroxyhydrocarbyle.

8. Composition selon la revendication 7, dans laquelle dans au moins environ 80% des amines, R' est choisi parmi les groupes n-pentyle, 3-méthylbutyle et 1-méthylbutyle, et R est choisi parmi les groupes n-heptyle, 3-méthylhexyle, 5-méthylhexyle, 2-méthylhexyle et 2-éthylpentyle; et R'' et R''' sont des groupes alkyle ou hydroxyalkyle.

9. Composition selon la revendication 7, dans laquelle 40 à 60% en poids des amines ont un substituant alkyle choisi parmi: 2-(3-méthylbutyl)-5-méthyloctyle, 2-(1-méthylbutyl)-5-méthyloctyle et 2-pentyl-5-méthyloctyle.

10. Composition selon la revendication 7, dans laquelle 65 à 80% en poids des amines sont constituées des amines de la revendication 7, en même temps que des amines ayant les substituants 2-(3-méthylbutyl)-7-méthyloctyle, 2-pentylnonyle, 2-pentyl-7-méthyloctyle et 2-(3-méthylbutyl)nonyle.

11. Composition selon soit la revendication 1 soit la revendication 3, dans laquelle la plupart des amines ont un substituant alkyle représenté par la structure:

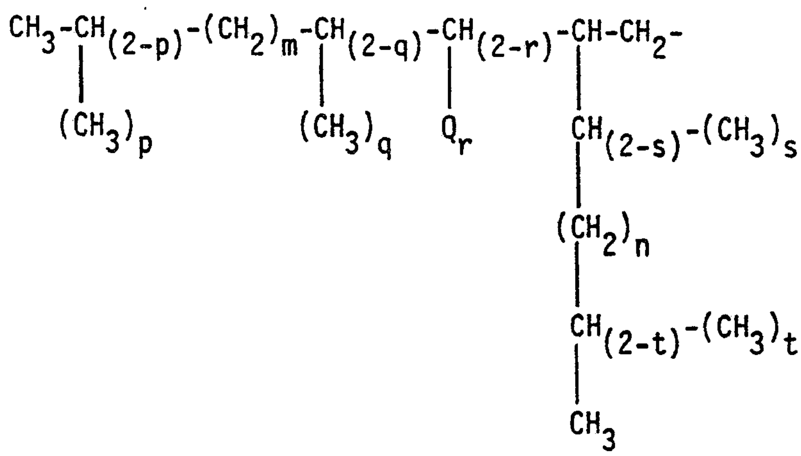

dans laquelle:

Q = groupe méthyle ou éthyle;
p, q et r = 0 ou 1, mais seulement l'un de p, q et r peut être 1;
m = 3, lorsque p, q et r = 0;
m = 2, lorsque r = 1 et Q = un groupe méthyle;
m = 1, lorsque r = 1 et Q = un groupe éthyle;
n = 2, lorsque s et t = 0;
n = 1, lorsque s ou t = 1;
s et t = 0 ou 1, mais seul l'un de s et t peut être 1.

12. Mélange d'amines isomériques selon soit la revendication 1, soit la revendication 3, et

caractérisé en ce que la plupart des amines de mélange comportent un groupe alkyle représenté par la structure:

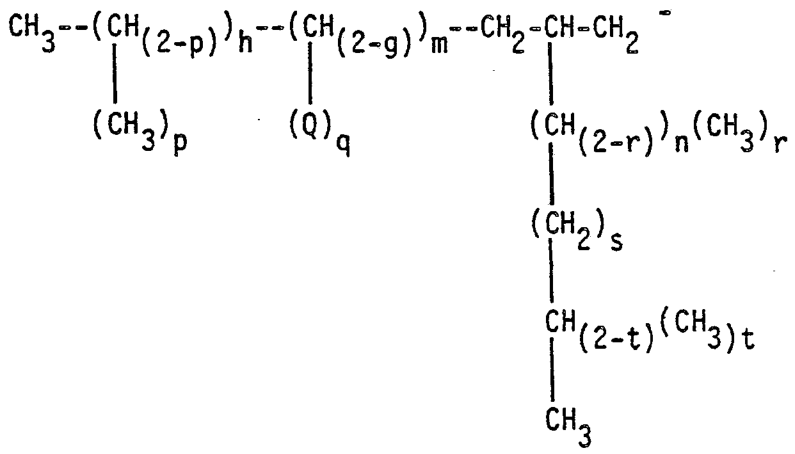

dans laquelle:

Q = un groupe méthyle ou éthyle; h et m = 0, 1, 2, 3, 4 et 5;
n et s = 0, 1, 2; p, q, r et t = 0 ou 1;
m + h ne peut jamais être égal à plus de 5
q et p = 0 lorsque m + h = 5 ou lorsque m + h = 3
h = 3 et m = 1 lorsque Q = un groupe méthyle et q = 1 et p = 0;
q = 0 et p = lorsque h = 2 et m = 2 ou lorsque h = 1 et m = 3;
n et s ne peuvent être jamais égaux à plus de 3;
r et t = 0 lorsque n + 3 ou lorsque n + s = 1;
s = 0 lorsque n, r et t = 1 ou lorsque n = 2 et r = 0 et t = 1;
s = 1 lorsque n = 1 et t et r = 0 ou 1 mais seulement l'un de t et r peut être 1;
s = 2 lorsque t = 1 et n = 0;
h = 2 et m = 1 lorsque Q = un groupe éthyle et q = 1 et p = 0.

13. Composition comprenant des dérivées oxydes d'amines ou de sels d'ammonium quaternaire d'un mélange d'amines tel que défini dans l'une quelconque des revendications précédentes.

14. Procédé de preparation d'un mélange d'amines ayant un substituant N-alkyle en $C_{10}$ à $C_{18}$ à partir d'une charge d'oléfines, qui comprend les étapes consistant à faire réagir une oléfine en $C_4$ à $C_8$ avec de l'oxyde de carbone et de l'hydrogène pour obtenir des aldéhydes en $C_5$ à $C_9$, à soumettre ces aldéhydes à une réaction d'aldolisation catalysée par les bases afin de produire des aldéhydes produits d'aldolisation en $C_{10}$ à $C_{18}$, et à faire réagir ces aldéhydes produits d'aldolisation avec une amine ou l'ammoniac dans des conditions d'alkylation réductrice pour produire des amines ayant un substituant N-alkyle en $C_{10}$ à $C_{18}$.

15. Procédé selon la revendication 14, dans lequel les aldéhydes produits d'aldolisation sont mis à réagir avec une amine pour produire des amines tertiaires avec un substituant alkyle en $C_{10}$ à $C_{18}$.

16. Procédé soit de la revendication 14 soit de la revendication 15, dans lequel la réaction formant les amines est effectuée sous pression d'hydrogène en contact avec un catalyseur pour réduction.

17. Procédé selon la revendication 16, dans lequel le catalyseur est un catalyseur en métal ou en oxyde métallique.

18. Procédé selon la revendication 16, dans lequel le catalyseur est un métal noble.

19. Procédé selon l'une quelconque des revendications 14 à 18, dans lequel le produit d'aldolisation produit est mis à réagir avec une dialkylamine dans laquelle les groupes alkyles ont chacun 1 à 4 atomes de carbone.

20. Procédé selon l'une quelconque des revendications 14 à 19, dans lequel les aldéhydes en $C_5$ à $C_9$ sont constitués d'au moins 50% d'alcanals sans ramification à la position 2 et le produit d'aldolisation ne contient pas plus de 20% de celui produit par l'aldolisation croisée d'aldéhydes 2-substitués.

21. Procédé selon l'une quelconque des revendications 14 à 19, dans lequel le produit d'aldolisation est mis à réagir sous forme d'énal.

22. Procédé selon l'une quelconque des revendications 14 à 21, dans lequel la réaction d'aldolisation est effectuée dans un alcali aqueux et il y a présent un cosolvant qui aide à améliorer le contact des aldéhydes avec l'alcali aqueux.

23. Procédé selon la revendication 22, dans lequel un composé hydroxyle est employé comme cosolvant.

24. Procédé selon l'une quelconque des revendications 14 à 23, dans lequel le produit d'aldolisation est hydrogéné pour donner des aldéhydes saturés et alors mis à réagir pour produire des amines.

25. Procédé selon l'une quelconque des revendications 14 à 24, dans lequel la réaction oxo est

effectuée avec un catalyseur de cobalt à une température comprise entre 80 et 150°C et une pression suffisante pour maintenir la stabilité du catalyseur mais non supérieure à 34,475 kPa, et la réaction d'aldolisation est conduite dans un milieu alcalin aqueux à une température comprise entre 90 et 130°C.

26. Procédé selon la revendication 19, dans lequel des aldéhydes pour la réaction d'aldolisation ont une teneur en alcanals sans ramification en position 2 de 60 à 80%, et la transformation de ces aldéhydes en produit d'aldolisation est comprise entre 75 et 90%.

27. Procédé selon la revendication 14 pour préparer des amines ayant des groupes hydrophobes dans l'intervalle des détergents, qui comprend les étapes consistant à conduire une réaction oxo avec l'hydrogène, l'oxyde de carbone et des oléfines choisies parmi celles en $C_5$ à $C_7$ afin d'obtenir des aldéhydes ayant de 6 à 8 atomes de carbone et constitué d'au moins 50% d'alcanals sans ramification à la position 2 et à soumettre les aldéhydes a une réaction d'aldolisation pour provoquer une bonne conversion en produit d'aldolisation mais celui préparé par aldolisation croisée d'aldéhydes 2-substitués étant constitué d'une proportion du produit et d'aldéhydes produits d'aldolisation réactifs non supérieure à 20% avec une amine ou $NH_3$ dans des conditions réductrices pour produire des amines ayant un substituant N-alkyle correspondant à l'aldéhyde produit d'aldolisation.

28. Procédé selon la revendication 27, dans lequel la réaction d'aldolisation est effectuée dans un alcali aqueux et il y a présent un cosolvant qui aide à améliorer le contact des aldéhydes avec l'alcali aqueux.

29. Procédé selon la revendication 14 por préparer des amines ayant une teneur élévée en substituants N-alkyle dans l'intervalle de $C_{11}$ à $C_{14}$, qui comprend les étapes consistant à conduire une réaction d'aldolisation du butanal avec des heptanals obtenus par la réaction oxo d'hexènes ramifiés provenant de la dimérisation du propylène, et à obtenir un produit d'aldolisation ayant une teneur élevée en 2-undécénals et à soumettre l'aldéhyde produit d'aldolisation à une réaction avec une amine dans des conditions d'alkylation réductrice.

30. Procédé selon la revendication 29 pour préparer des amines ayant des substituants alkyle en $C_{14}$ qui comprend les étapes consistant à effectuer une réaction oxo avec de l'hydrogène, de l'oxyde de carbone et un mèlange d'hexènes qui se compose principalement de méthylpentènes avec une insaturation interne avec pas plus de 30% d'hexènes linéaires de manière à obtenir des aldéhydes en $C_7$ avec moins de 30% d'aldéhydes 2-substitués et à soumettre les aldéhydes à une réacton d'aldolisation afin d'obtenir des aldéhydes produits d'aldolisation avec une conversion d'au moins 70% et à faire réagir un aldéhyde produit d'aldolisation avec une dialkyl amine dans des conditions réductrices pour produire une amine substituée par un groupe alkyle en $C_{13}$.

31. Procédé selon l'une quelconque des revendications 14 à 30, dans lequel la réaction avec une amine est à une température non supèrieure à 50°C.

32. Procédé selon la revendication 31, dans lequel la réaction avec l'amine utilise un catalyseur de palladium sous pression d'hydrogène.

33. Procédé selon la revendication 27, dans lequel la réaction avec une amine s'effectue à une température comprise entre 35 et 80°C.

34. Procédé selon la revendication 30, dans lequel le mélange d'hexènes comprend sensiblement le mélange d'hexènes provenant d'une dimérisation du propylène sur un catalyseur de nickel et d'aluminium alkyl.